# EUROPEAN PATENT SPECIFICATION

(11) **EP 2 988 704 B1**
(45) Date of publication and mention of the grant of the patent: **16.01.2019**
(21) Application number: 14788149.4
(22) Date of filing: 23.04.2014
(51) Int. Cl.: A61F 2/915

(54) **EXPANDABLE DEFORMABLE SLIDE AND LOCK STENT**
DEHN- UND VERFORMBARER GLEIT- UND VERSCHLUSSSTENT
ENDOPROTHÈSE DÉFORMABLE EXTENSIBLE À GLISSIÈRE ET VERROU

(30) Priority: 25.04.2013 US 201361815854 P; 27.04.2013 US 201361816724 P
(43) Date of publication of application: 02.03.2016
(73) Proprietor: Reva Medical, Inc., San Diego, CA 92111 (US)
(72) Inventor: WEIER, Keith, San Diego, CA 92110 (US); DENNIS, Robert, F., San Diego, CA 92103 (US)
(74) Representative: Zacco Sweden AB
(86) International application number: PCT/US2014/035183
(87) International publication number: WO 2014/176361

(56) References cited:
- WO-A1-2007/016409
- WO-A1-2010/022005
- US-A- 5 843 175
- US-A- 6 066 169
- US-A- 6 123 721
- US-A1- 2010 131 048
- US-A1- 2011 230 956
- US-B1- 6 375 677

## Description

### CROSS-REFERENCE TO RELATED APPLICATIONS

This Application claims from the benefit of US Application No. 61/815,854, filed April 25, 2013, and of US Application No. 61/816,724, filed April 27, 2013. In certain respects, this Application is related in subject matter to US Application No. 13/083,508 filed April 8, 2011, now US Patent No. 8,523,936, which claims priority to US Application No. 61/322,843, filed April 10, 2010. In some respects, this Application is also related in subject matter to US Patent No. 7,947,071.

### BACKGROUND

### Field

The present disclosure relates generally to expandable medical implants for maintaining support of a body lumen, such as a stent having improved mechanical and post-deployment dynamic capabilities.

### Description of Certain Related Art

Various embodiments of vascular implants (such as stents, thrombus filters, and heart valves) are used in their various embodiments for medical applications. Of these vascular devices, one of the leading candidates as a stent device and structural component is the radially expandable and slidably engaged stent as disclosed in US Patent Nos. 8,292,944; 8,277,500; 7,988,721; 7,947,071; 7,704,275; 7,914,574; 6,033,436; 6,224,626; and 6,623,521.

Other radially expandable and slideably engaged stents, such as those disclosed in US Patent Nos. 5,797,951; 5,549,662; and 5,733,328, further describe the state of the art.

The art includes various embodiments of stents that are expandable, such as described in US Patent and Publication Nos. 6,468,302; 5,741,327; 5,102,417; 5,514,154; 2007-0253,996; 2007-0283,552, and the like. In particular, WO2007016409 discloses a radially expandable stent having a plurality of rings arranged longitudinally to form a tubular body assembly.

### SUMMARY

Although promising candidates for use as implantable devices and device components, certain known radially expandable and slidably engaged stents have mechanical and vasodynamic limitations. These limitations can be characterized as deployment related limitations, and limitations related to vasodynamic capabilities.

Intravascular space, especially that of a patient in need of a vascular implant, is generally inconsistent and varies upon the individual with respect to curvature, plaque buildup and other luminary obstructions. Procedures are available to physicians such as balloon angioplasty, which aid in the reduction of plaque prior to stenting. However, even after such procedures, vascular characteristics remain patient delineated and largely inconsistent. Inconsistencies in vascular characteristics; such as the interference due to a luminary occlusion, require flexibility, distribution of material strength, and vascular adaptability of devices to be implanted.

A vascular implant may experience a number of mechanical limitations related to delivery. For example, some portions of the vasculature are curved or substantially non-cylindrical. These portions of the vasculature have proven difficult to deploy stent devices. Sometimes, the curvature of the vessel can cause a deployed stent to fold, especially in stents with insufficient flexibility in the design.

A vascular implant may also experience a number of countering forces post-deployment. Some of these countering forces are a result of what is herein referred to as vasodynamics; the resulting movements, constrictions and contortions of the vasculature. Of these countering forces is crush force, caused by post-expansion elastic recoil of the vessel.

Additionally, some stents experience an occlusion-derived impaction force (e.g., a point force derived from the impact of a luminary occlusion directly onto the device). For example, such a luminary occlusion can be plaque or a thrombus. Other countering forces such as dilation, and contortion, are to be discussed in further detail below.

Many devices are fabricated from a biodegradable polymer which may become substantially more ductile and flexible with the progression of time up to a point of water absorption equilibrium. As water is absorbed, the polymer material becomes bendable or ductile. Differing polymer compositions will have a varied rate of moisture absorption. The present application recognizes the benefits of controlled water absorption into the polymer material such as a reduced propensity for micro fissures.

The present application recognizes that certain embodiments of a mechanically-improved stent design will overcome the limitations set out above, or others. Certain embodiments of the mechanically-improved stent design can increase adaptability to the dynamics of the vasculature.

Many prior art stent embodiments are designed around crush force and maintaining patency of a luminary space. Although patency of the lumen is of concern, there are other factors to be addressed in an effort to go beyond bare functionality, so as to move toward successful treatment and healing of a vessel. For example, other concerns can be whether the stent is adapted to accommodate changes in the size and/or shape of the vasculature.

The vasculature is a dynamic system. Although it is difficult to quantify, the vasculature may experience a number of dynamic movements at any given moment in time. Of these is a wave-like dilation, which presents variability in the interior diameter of the vessel at a given location. Dilation can occur from a change in blood pressure or other change in the circulation. Additionally, portions of the vasculature can experience a contortion or twist like motion in addition to dilation.

Where there is plaque or a luminary occlusion, the vasculature can experience a resistance to these natural movements. Such a resistance can cause the adjacent tissue to undergo a cytotic response, such as the division of cells, or intravascular cell growth known as neointimal growth. Neointimal growth is a new or thickened layer of arterial intima formed especially on a prosthesis or in atherosclerosis by migration and proliferation of cells.

Clinical data generally shows that stent implants stimulate neointimal growth in the vessel immediately subsequent to implantation. Neointimal growth is acceptable up to a point where blood pressure is substantially increased or where the lumen is obstructed and blood can no longer efficiently pass.

It is thought that resistance to vasodynamics, among other things, can dramatically increase stenosis surrounding an implanted vascular device. Therefore, it can be helpful to understand the dynamics of the vasculature and to design a stent capable of maintaining patency of the lumen while promoting the motions associated with vasodynamics such as periodic dilation and contortion. A stent designed to incorporate the dynamics of the vasculature can better serve to treat and ultimately heal the vessel. For example, because neointimal growth typically surrounds and encompasses the implanted stent, leaving the stent to reside substantially within the new vessel wall, it can be advantageous for the stent to be adapted to minimize further stenosis in that state.

Although stents can be made of generally any biocompatible material, there is a movement toward the use of stents fabricated from a biodegradable and bioresorbable polymer. Biodegradation is the structural decomposition of a polymer, often occurring as bulk erosion, surface erosion, or a combination thereof. Bioresorption includes the cellular metabolism of the degraded polymer.

Insofar as the terms "invention" and/or "embodiment" are used in the following, and/or features are presented as being optional, this should be interpreted in such a way that the only protection sought is that of the invention as claimed. The present application describes a stent that, in some embodiments, is capable of utilizing the degradation and resorption properties of the polymer to enhance the healing and treatment of the vessel.

The present disclosure also describes a stent that, in some embodiments, has a rotationally flexible backbone capable of adaption to vasodynamic movements, thereby minimizing stenosis of the vasculature.

The present disclosure also describes a stent that, in some embodiments, is capable of being efficiently encapsulated with neointimal growth. In certain variants, initial degradation of the stent material will transform the stent into a rotationally flexible and vaso-adaptive support within the new vessel wall.

The present disclosure also describes a stent that, in some embodiments, includes a slide and lock mechanism or other device to facilitate expansion of the stent. For example, the stent can be expandable via deformable members (e.g., plastically deformable struts or shape memory structures). Example embodiments comprise tubular deformable structures integrated with slide and lock mechanisms, which provide additional resistance to recoil following deployment.

The present disclosure also describes various embodiments of expandable stents. In certain embodiments, the stents provide radial support to maintain patency of a lumen, a flexible vaso-adaptive backbone structure, and/or a uniform circumferential distribution of slideable engagements.

The present application also describes certain stent embodiments capable of solving the aforementioned problems, or others. For example, some embodiments can reduce or eliminate the aforementioned problem regarding restriction of vasodynamic movements.

Additionally, the present application describes a stent having, in some embodiments, flexibility sufficient to promote and adapt to natural vasodynamic movements (e.g., wave-like dilation and contortion movements) while maintaining patency of the lumen. In certain embodiments, such flexibility of the stent can reduce or minimize stenosis.

In some embodiments, a radially expandable stent has longitudinal and circumferential axes and includes a plurality of rings arranged longitudinally. This can form a tubular body assembly. Each ring can include a circumferential sequence of cells. In some implementations, each cell is longitudinally defined by a pair of deformable struts on a first longitudinal side and a second set of deformable struts on a second longitudinal side. The struts of the first pair can be set at an angle to one another and the struts of the second pair can be set at an angle to one another. The angle between each pair can change (e.g., increase) as the stent is expanded from a compact configuration towards an expanded configuration. In certain implementations, the stent includes a rail member.

In certain variants, at least one cell is circumferentially defined by a first cross member and a second cross member. The first cross member can fixedly support a distal end of the rail member. The second cross member can slidingly engage a medial portion of the rail member. In certain embodiments, the rail member slides circumferentially in a first direction with respect to the second cross member as the stent is expanded towards an expanded configuration.

In some embodiments, the medial portion of the rail member includes at least one tooth element configured to engage the second cross member, such as to permit sliding in the first direction. In some embodiments, the tooth element is configured to engage the second cross member so as to resist sliding in an opposite second direction. This can prevent or inhibit the stent from radially contracting.

In various embodiments, the stent includes one of, or any combination of, any of the following. The stent can include a plurality of rail members. In some embodiments, each cell is circumferentially defined by a first cross member and a second cross member. A plurality or each first cross member can fixedly support a distal end of one of the rail members. A plurality or each second cross member can slidingly engage a medial portion of one of the rail members.

The stent includes one of, or any combination of, any of the following. In some embodiments, the stent includes link elements connecting longitudinally adjacent rings. In some embodiments, the longitudinally adjacent rings have integrated portions (e.g., share common structural portions). In some variants, each of the rings has a substantially zig-zag shape. For example, the one or more of the rings can have a plurality of peaks and a plurality of valleys. In some embodiments, the rings are arranged in a peak-to-peak configuration.

According to some embodiments, a tubular stent with longitudinal and circumferential axes includes a radially-expandable first annular support and a radially-expandable second annular support. The first and second annular supports can each include a plurality of struts and can each have an undulating shape. The undulating shape can include a plurality of peaks and valleys. In certain implementations, the second annular support is about 180 degrees out of phase with the first annular support such that the first and second annular supports are arranged in a peak-to-peak configuration. In some implementations, the second annular support is about in-phase with the first annular support such that the first and second annular supports are arranged in a peak-to-valley configuration.

The stent can include a first cross member, such as a member connecting one of the peaks of the first annular member with one of the peaks of the second annular member. The stent can include a second cross member, such as a member connecting another one of the peaks of the first annular member with another one of the peaks of the second annular member. In some variants, the second cross member includes a channel.

Various embodiments include a rail member. The rail member can be anchored to the first cross member and can pass through the channel of the second cross member. The rail member can be configured to slide relative to the second cross member in a first circumferential direction, thereby facilitating radial expansion of the stent. Some embodiments of the stent include a locking mechanism on the rail member. The locking mechanism can be configured to engage the second cross member. This can prevent or inhibit the rail member from sliding relative to the second cross member in a circumferential direction generally opposite to the first circumferential direction, thereby inhibiting radial contraction of the stent.

In various embodiments, the stent includes one of, or any combination of, any of the following. The locking mechanism can include a plurality of teeth. The channel can include a closed aperture. The channel can be open on a radial side. In some embodiments, the peaks and valleys are substantially flat (e.g., substantially parallel with the longitudinal axis). The struts can be configured to rotate with respect to the longitudinal axis. In some variants, when the stent is in the compacted state, and when the stent is in the expanded state, the struts are not parallel with the longitudinal axis. In some variants, when the stent is in an intermediate state, between the compacted state and the expanded state, the struts are substantially parallel with the longitudinal axis.

In certain embodiments, the stent includes one of, or any combination of, any of the following. In some implementations, when the stent is in the compacted state, a given one of the struts is positioned at a first angle relative to a line parallel with the longitudinal axis. In some implementations, when the stent is in the expanded state, the given one of the struts is positioned at a second angle relative to the line parallel with the longitudinal axis, the second angle being greater than the first angle.

In certain embodiments, an expandable slide and lock stent is a generally tubular member having a circumferential and longitudinal axes. The stent can include a bond backbone and a slot backbone. The first and second backbones can extend along the longitudinal axis. The bond and the slot backbones can each having an undulating shape having a plurality of peaks. The bond and the slot backbones can be in a corresponding arrangement such that the peaks of the bond and the slot backbones are substantially longitudinally aligned. This can facilitate nesting of the backbones when the stent is in the compacted state.

Some embodiments include a bond area on the bond backbone. The bond area can be located in one of the peaks of the bond backbone. Some embodiments include a slot in the slot backbone. The slot can be substantially parallel with the circumferential axis. The slot can be located in the peak of the slot backbone that corresponds to the peak in the bond backbone in which the bond area is located.

In some embodiments, the stent includes an elongate rail member. The rail member can have a substantially linear shape and can include proximal and distal ends. The proximal end of the rail member can be connected with the bond area on the bond backbone. The distal end of the rail member can extend circumferentially from the bond backbone. The rail member can pass through the slot. The rail member can be configured to engage a locking mechanism with the slot in the slot backbone.

The rail member can be configured to slide relative to the slot backbone in a circumferential direction. The engagement between the locking mechanism and the slot can prevent or inhibit movement of the rail member in an opposite circumferential direction. This can provide one-way movement of the bond backbone away from the slot backbone, which can allow the tubular member to be expanded from a compacted diameter and an expanded diameter.

In various embodiments, the stent includes one of, or any combination of, any of the following. The stent can include a second rail member connected to a second bond area on the bond backbone and passing through a second slot in the slot backbone. The second rail member can extend in a circumferential direction generally opposite the direction of the first rail member. The bond and the slot backbones each further comprise plurality of valleys. The the second bond area being located in a valley of the bond backbone and the second slot being located in a corresponding valley of the slot backbone. The slot can include a closed aperture. The bond area can be open on a radial side. The peaks can be substantially parallel with the longitudinal axis. The locking mechanism can include a row of teeth on a first side and a second side of the rail member.

Any of the structures, materials, steps, or other features disclosed above, or disclosed elsewhere herein, can be used in any of the embodiments in this disclosure. Any of the structures, materials, steps, or other features that are shown and/or described herein can be used in combination with any other of the structures, materials, steps, or other features that shown and/or described herein.

### BRIEF DESCRIPTION OF THE DRAWINGS

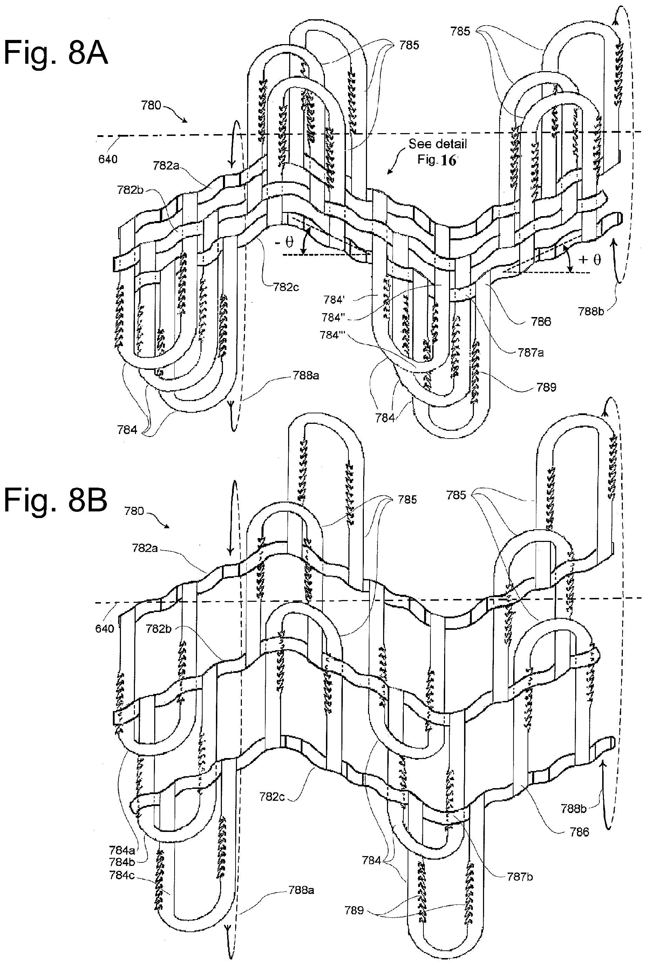

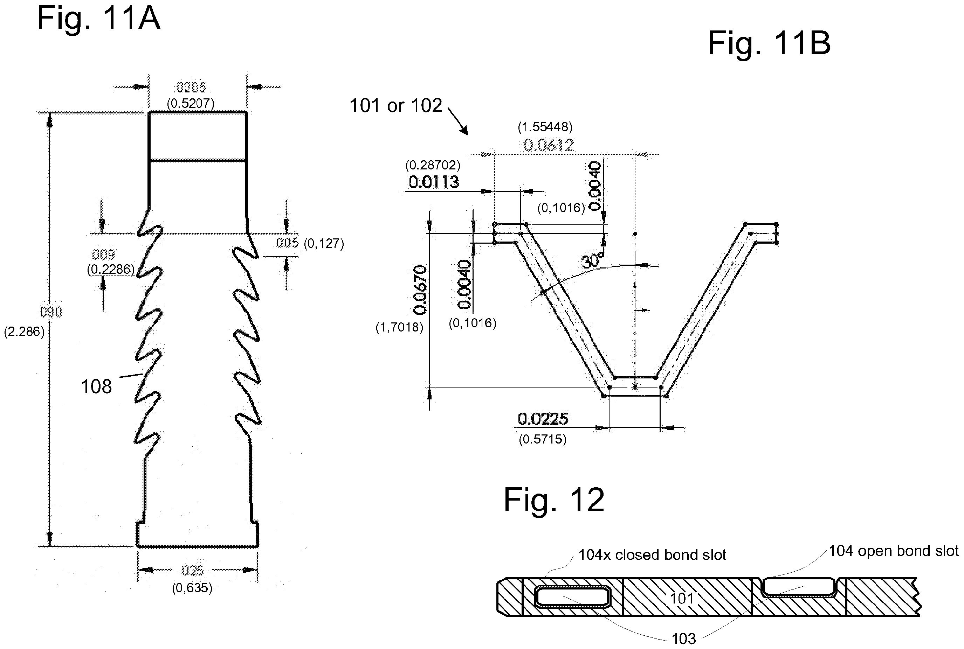

The abovementioned and other features of the embodiments disclosed herein are described below with reference to the drawings of the embodiments. The illustrated embodiments are intended to illustrate, but not to limit the embodiments. The drawings contain the following figures:
FIG. 1 is a planar representation of an embodiment of a tubular slide & lock stent assembly 100 in a compacted configuration, in which the lateral dimension represents the longitudinal axis of the stent, and the vertical dimension represents the circumferential direction of the tubular assembly, according to an embodiment. The planar surface representing the outside of the tubular shape, and the upper portion of the figure may be conceived as wrapping around into the page to join the bottom portion of the figure.
FIG. 2 is a planar representation of a tubular slide & lock stent assembly 100 of FIG.1, shown in a partially expanded configuration.
FIG. 3 is a planar representation of a tubular slide & lock stent assembly 100 of FIG.1, shown in a fully expanded configuration.
FIG. 4 is a planar representation of a portion of the assembly of FIGS. 1-3, in a partially expanded configuration similar to FIG. 2, and shows a repeatable longitudinal module comprising a pair of backbones with associated rail array.
FIG. 5 depicts a portion of an embodiment of a single rail member as included in the stent assembly 100, and details of its structure.
For purposes of comparison, and for illustrating certain features, FIGS. 6A and 6B are Figures 11A and 11B, respectively, of US Patent No. 8,523,936 (Application No. 13/083,508 filed April 8, 2011).
FIGS. 7A, 7B and 7C illustrate details of the stent assembly embodiment 100 shown in FIGS. 1-3. FIG. 7A is a detail of a portion of the planar array of FIG. 2. FIG. 7B is a cross-section of a portion of a bond backbone 101 showing details of bond channels 104. FIG. 7C is a cross-section of a portion of an engagement/locking backbone 102 showing details of pass-through or tooth engagement slots 105.
For purposes of comparison, and for illustrating certain features, FIGS. 8A and 8B are Figures 12A and 12B, respectively, of US Patent No. 8,523,936 (Application No. 13/083,508 filed April 8, 2011). These figures illustrate an alternative tubular stent assembly having rail modules with rail members in planar representation in compacted and expanded configurations respectively.

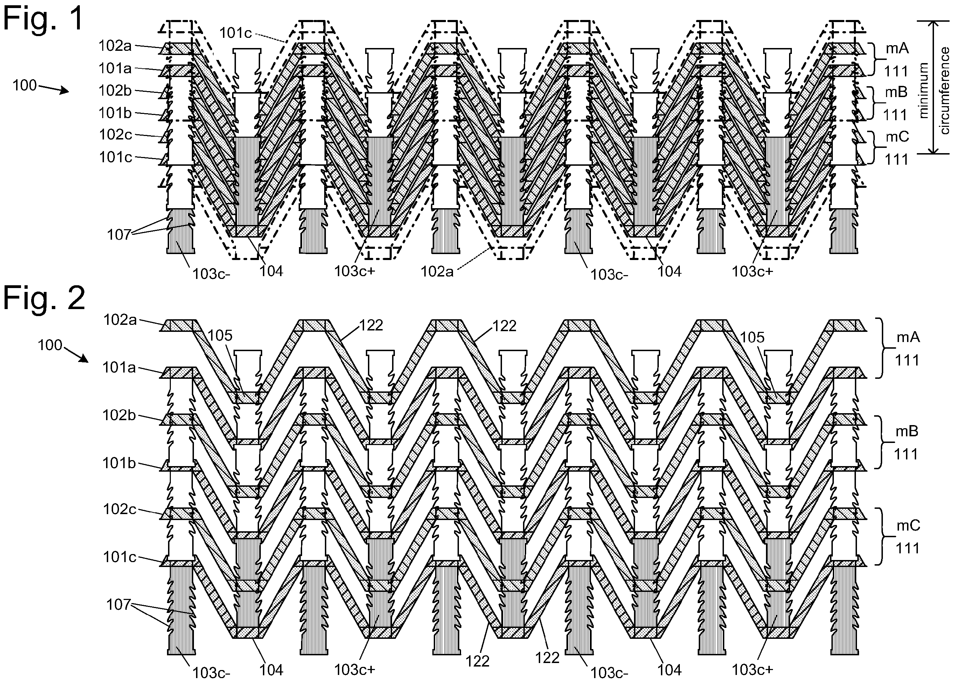

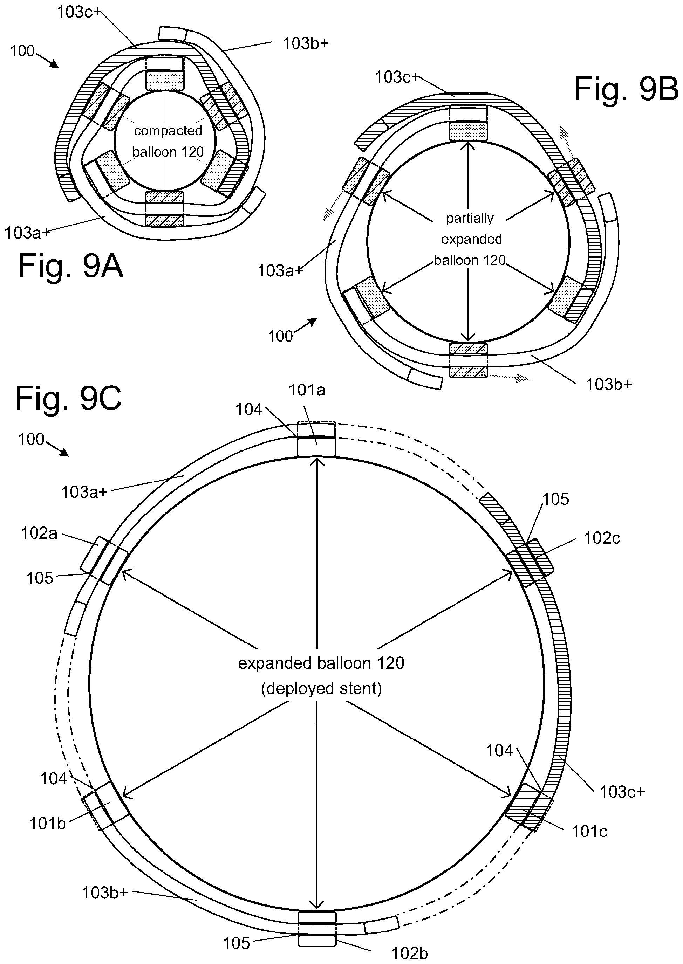

FIGS. 9A-9C illustrate the stent of FIGS. 1-3 mounted on an expandable member, such as a balloon. In FIGS. 9A-C, the tubular stent assembly 100 is depicted end-on, from a perspective view along the stent longitudinal axis.

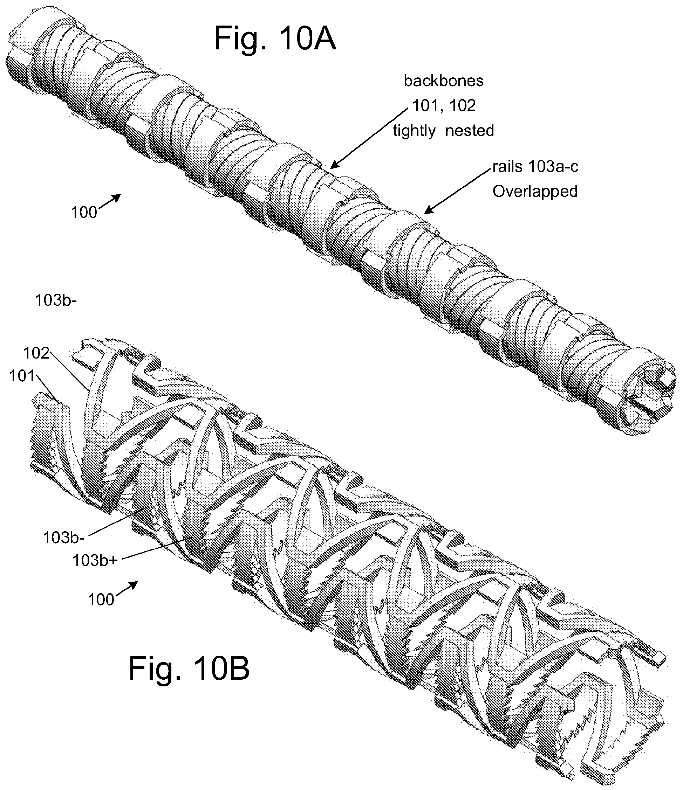

FIGS. 10A and 10B illustrate isometric perspective view of the stent 100, in compacted and expanded configurations.
FIGS. 11A and 11B show illustrative examples of dimensions and angles of the backbones 101 or 102, and rails 103.
FIG. 12 shows a configuration of the bond channel 104, indicated as slot 104x, in which the slot 104x is enclosed by the backbone structure.

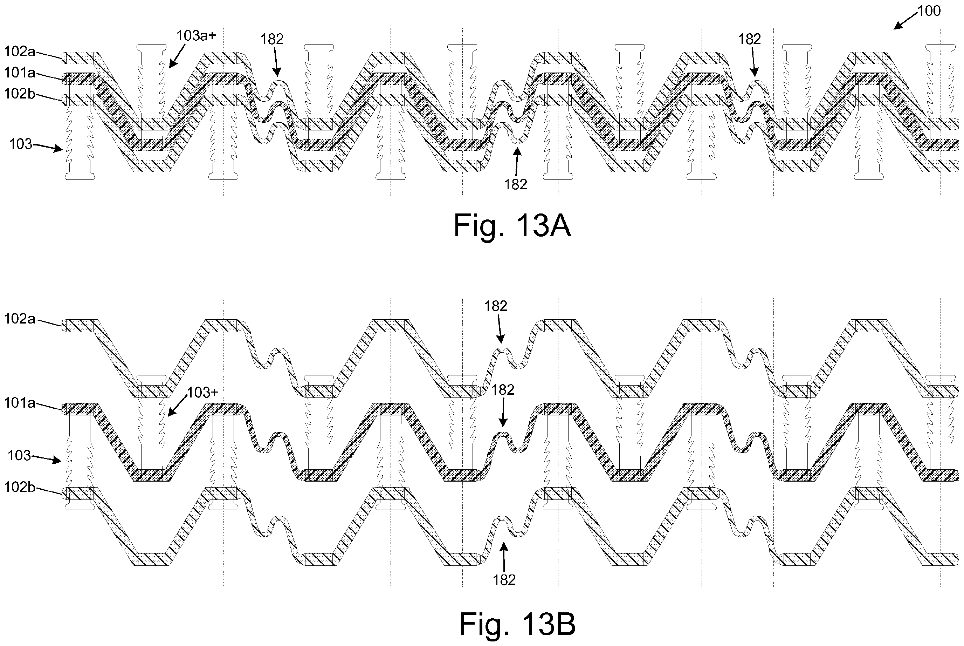

FIGS. 13A and 13B show embodiments in which the bond backbone and/or the slot backbone include one or more link elements.

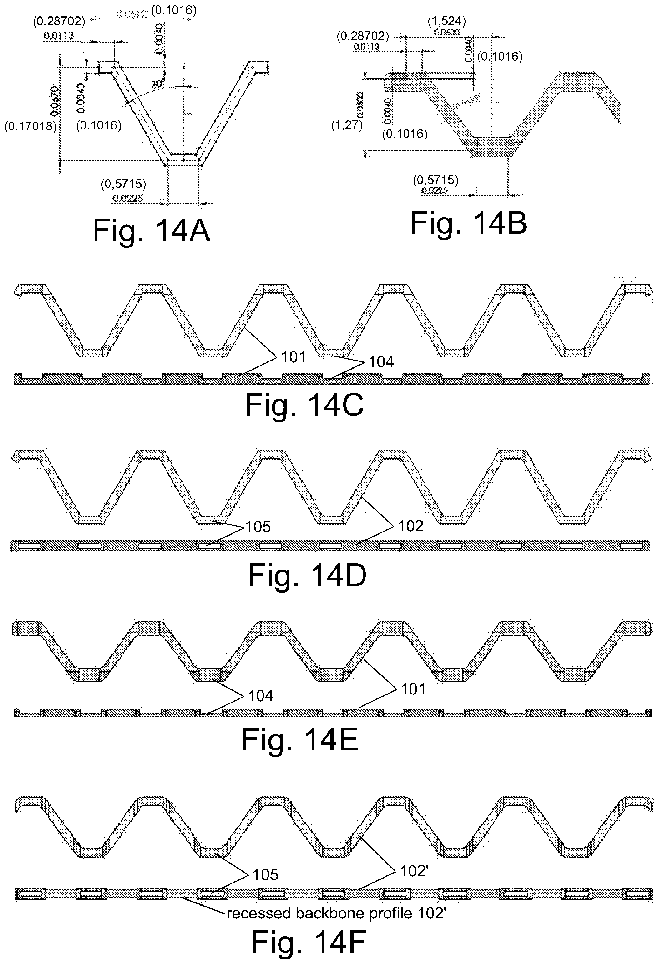

FIGS. 14A-F shows a number of top and side views of certain backbone configurations, including a backbone having a recessed profile between slots.

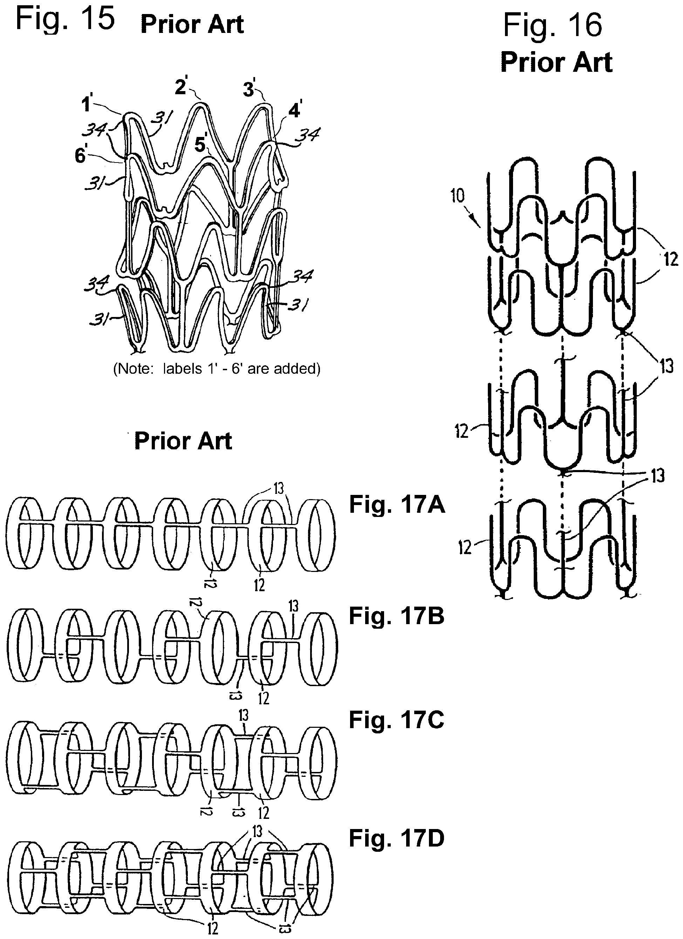

FIGS. 15, 16, 17A-D, 18, and 19 are Figures 13, 4, 7-10, 11, and 5, respectively, from US Patent No. 5,514,154, with certain rearrangements for presentation purposes and showing certain aspects.

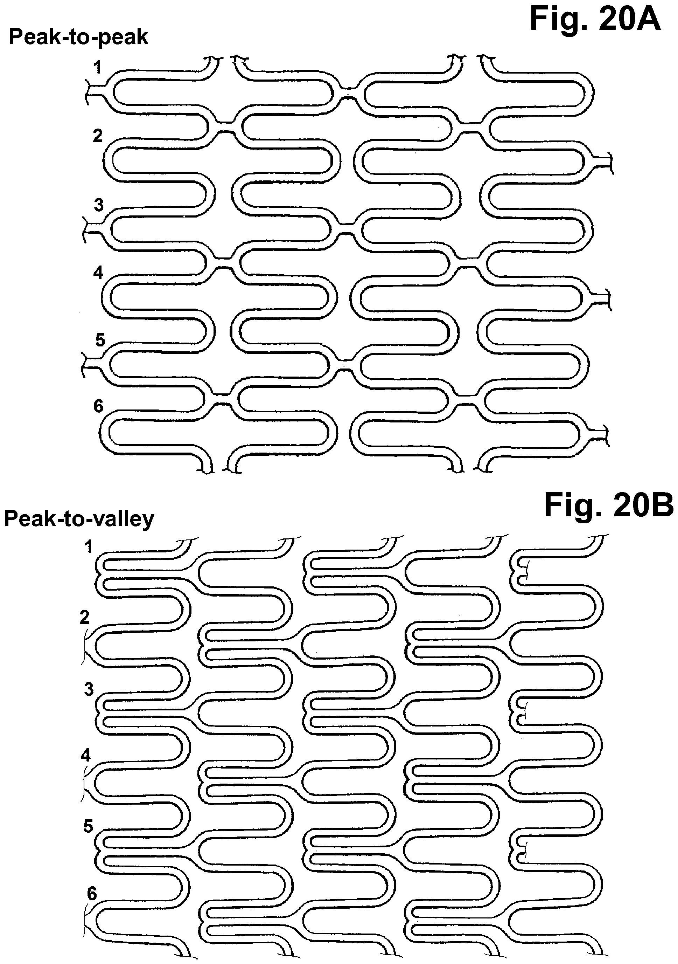

FIGS. 20A and 20B show examples of tubular arrays of ring structures, which can be suitable for expandable stent embodiments.

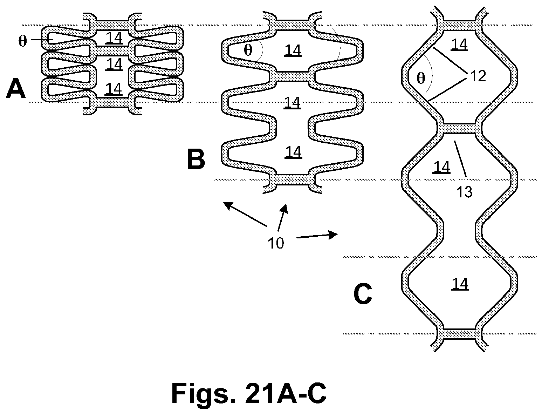

FIGS. 21A-C illustrate the compacted to expanded configurations of a stent sub-structure comprising a plurality of deformable struts connected to one another as circumferential rings, and in which two such rings are cross-linked to form a sequence of cells in circumferential arrangement.

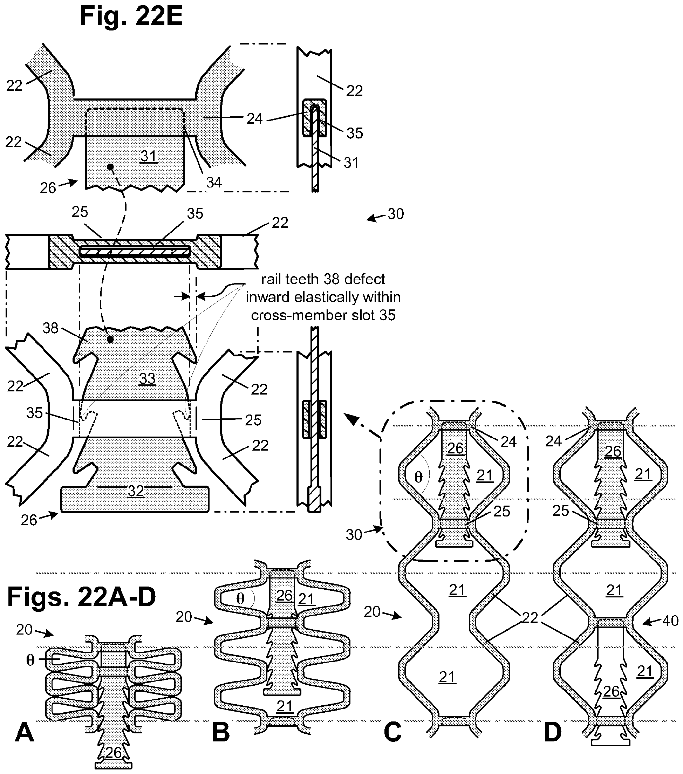

FIGS. 22A-D illustrate compacted to expanded configurations of a stent sub-structure comprising a circumferential ring of cells similar to those shown in FIGS. 21A-C, with selected cells connected to a slide and lock mechanism (S&L) to resist recoil. FIGS. 22A-C illustrate an example embodiment having a spacing of one S&L mechanism for each three sequential cells of the ring. FIG. 22D illustrates an embodiment having a closer spacing of one S&L mechanism for each two sequential cells of the ring.
FIG. 22E is a multi-view illustration of the details of the S&L mechanism of FIGS. 22A-D.

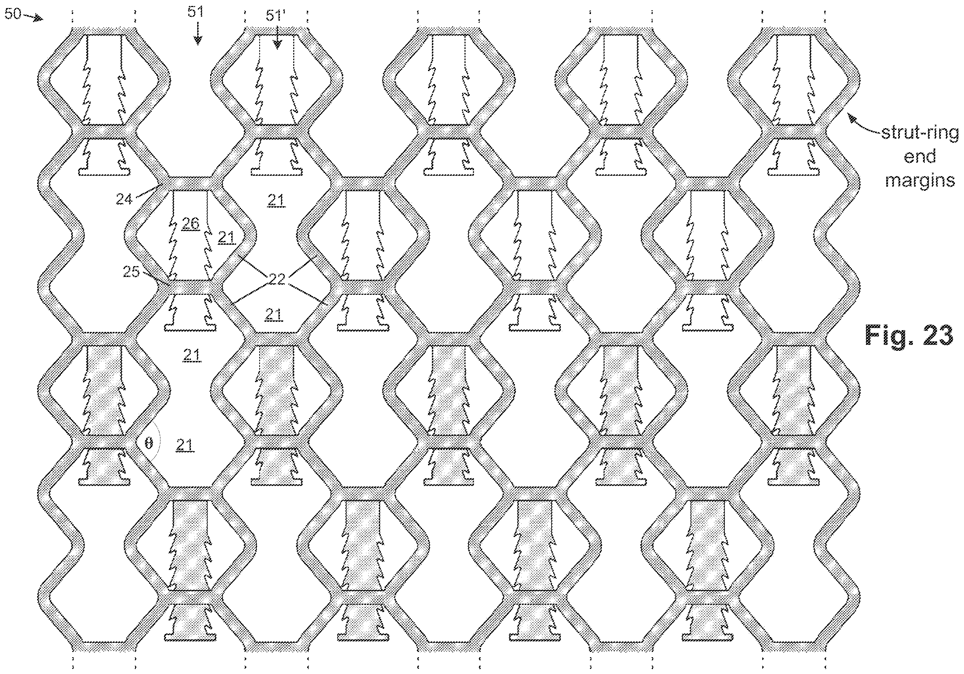

FIG. 23 illustrates an embodiment of a tubular stent structure, in which circumferential rings of cells having spaced-apart S&L mechanisms bracing certain ones of the cells (S&L braced cells). Adjacent rings of cells can be integrated so that several and/or each ring (e.g., except end rings) forms part of two adjacent rings of cells.

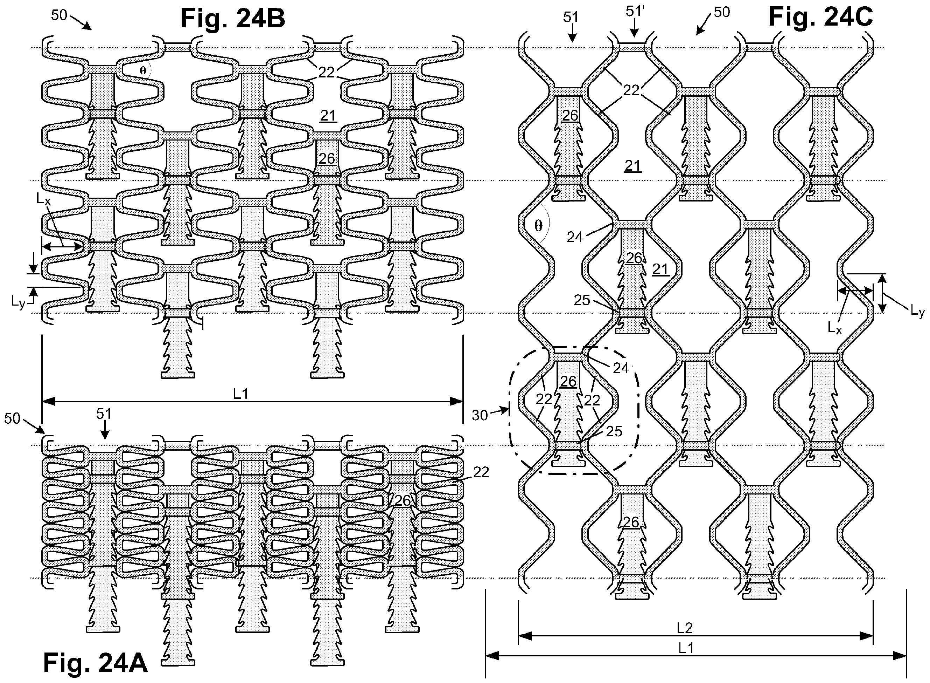

FIGS. 24A-C illustrate compacted to expanded configurations of an embodiment of a stent structure, which can be generally similar to FIG. 23 in some respects.

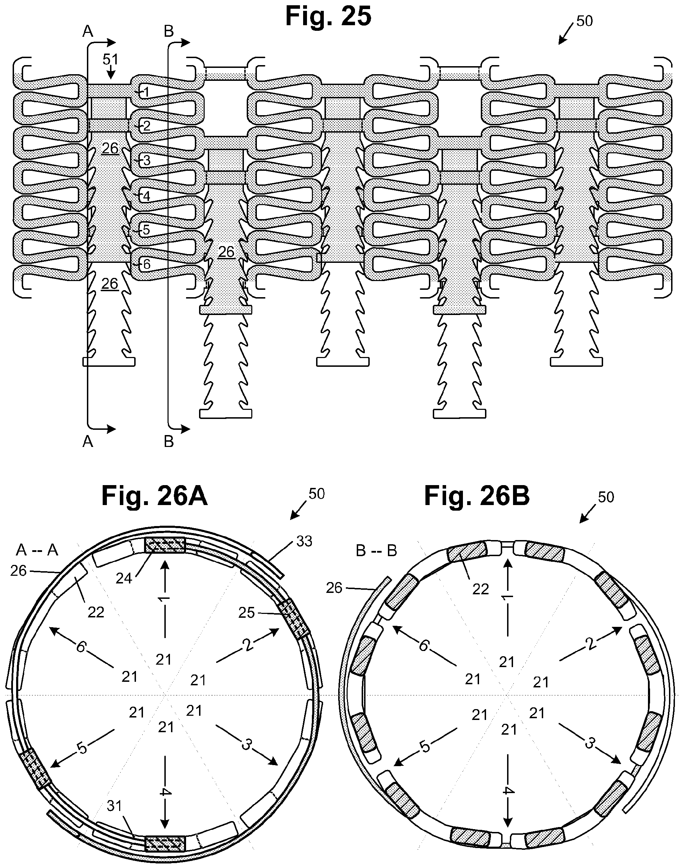

FIGS. 25 and 26A-B illustrate certain details of the compacted structure of FIG. 24A. FIG. 25 illustrates the stent of FIG. 24A as a planar representation for purposes of presentation. FIG. 26A shows a cross-section of the tubular stent along line A-A in FIG. 25. FIG. 26B shows a cross-section of the tubular stent along line B-B in FIG. 25.

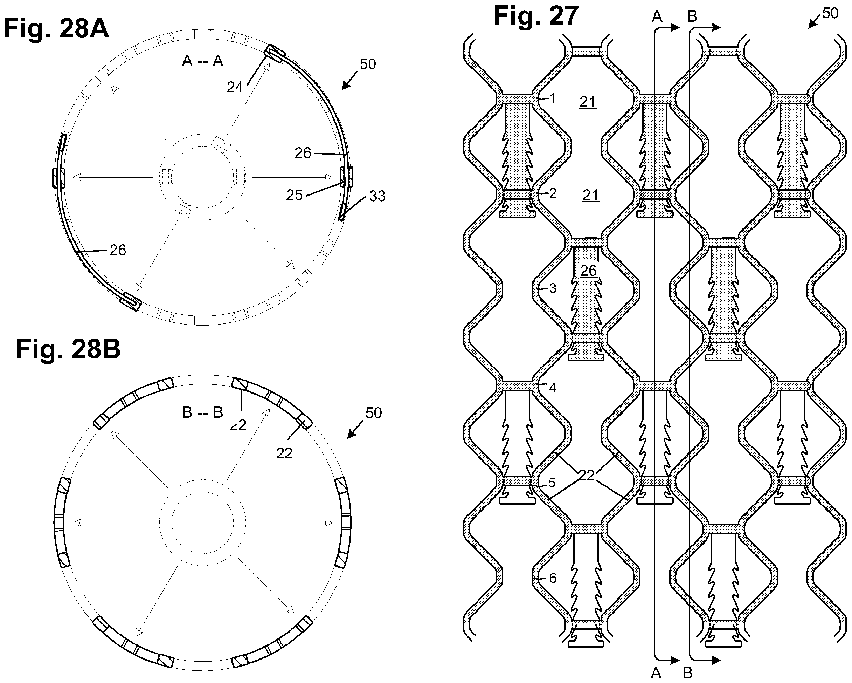

FIGS. 27 and 28A-B illustrate certain details of the expanded structure of FIG. 24C. FIG. 27 illustrates the stent of FIG. 24C as a planar representation for purposes of presentation. FIG. 28A shows a cross-section of the tubular stent along line A-A in FIG. 27. FIG. 28B shows a cross-section of the tubular stent along line B-B in FIG. 27.

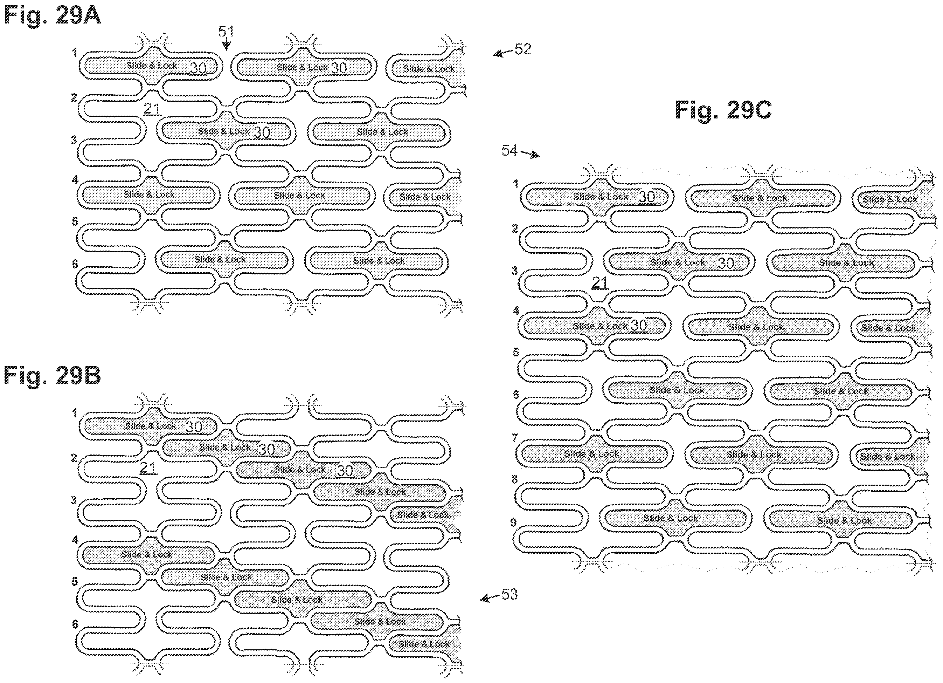

FIGS. 29A-C show three examples of the many possible alternative configurations of S&L braced deformable stent structures. These examples have a spacing of S&L braced cells of 1:3, as in FIG. 22C.

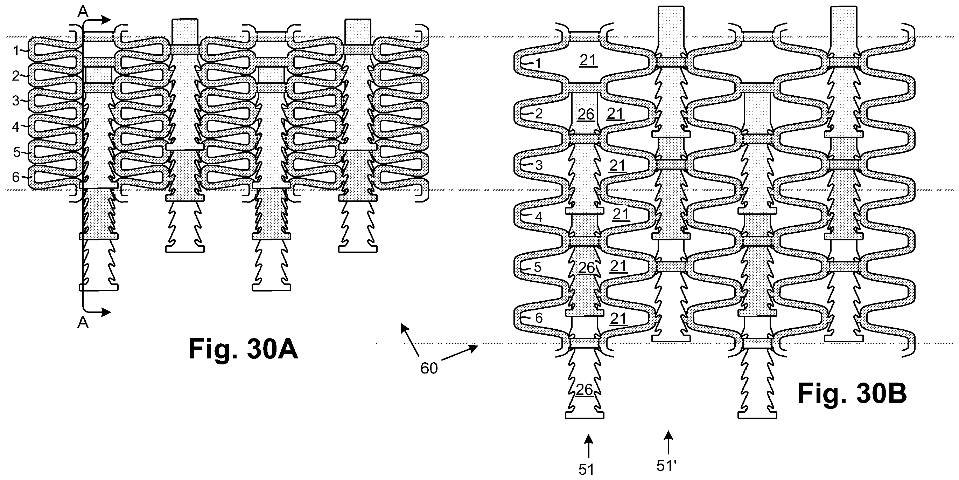

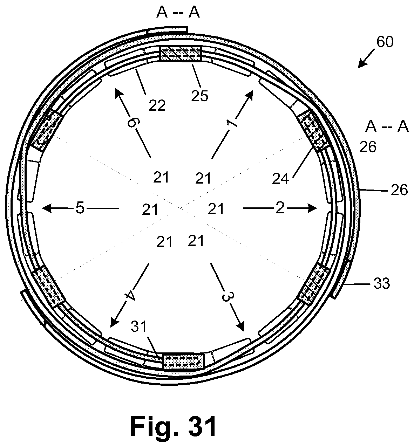

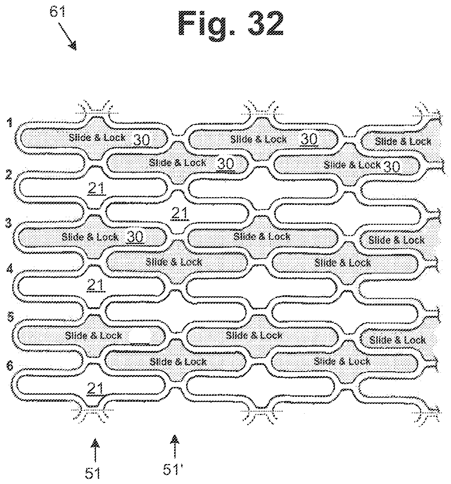

FIGS. 30A-C, 31, and 32 illustrate an embodiment of a tubular stent structure, generally similar to the example of FIGS. 24-27 in some respects. The illustrated embodiment includes a spacing of S&L braced cells of 1:2 (e.g., one S&L mechanism for each two sequential cells of the ring), as in FIG. 22D. FIGS. 30A-C illustrate the compacted to expanded configurations of an embodiment of a stent structure. FIG. 31 shows a cross-section of the tubular stent along line A-A in FIG. 30A. FIG. 32 illustrates that adjacent S&L mechanisms can be arranged in a staggered or alternating pattern.

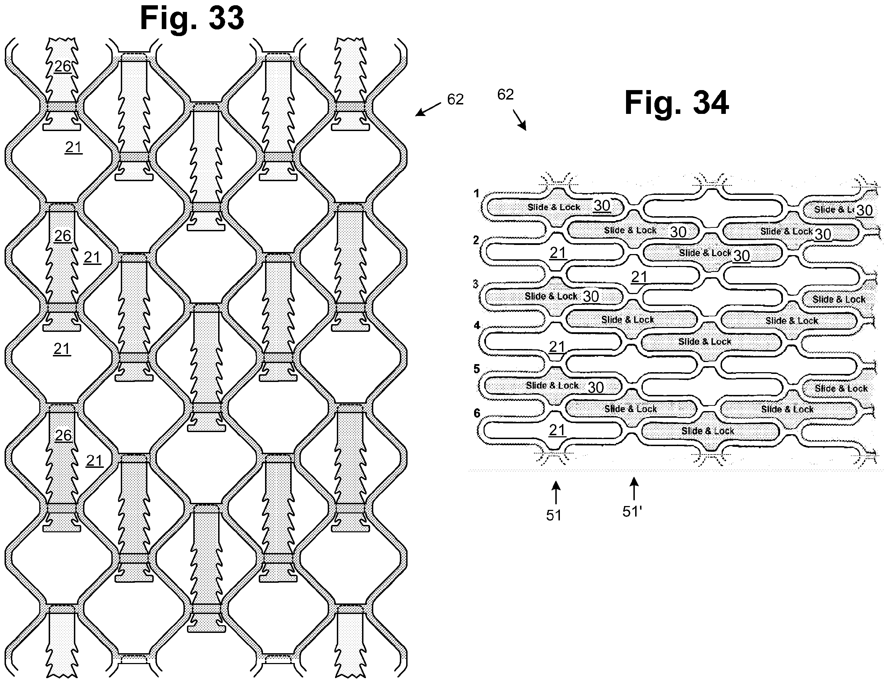

FIGS. 33 and 34 illustrate an arrangement of the S&L braced cells in an embodiment having 1:2 spacing, wherein adjacent S&L mechanisms are arranged in an undulating (e.g., a zig-zag or chevron) pattern.

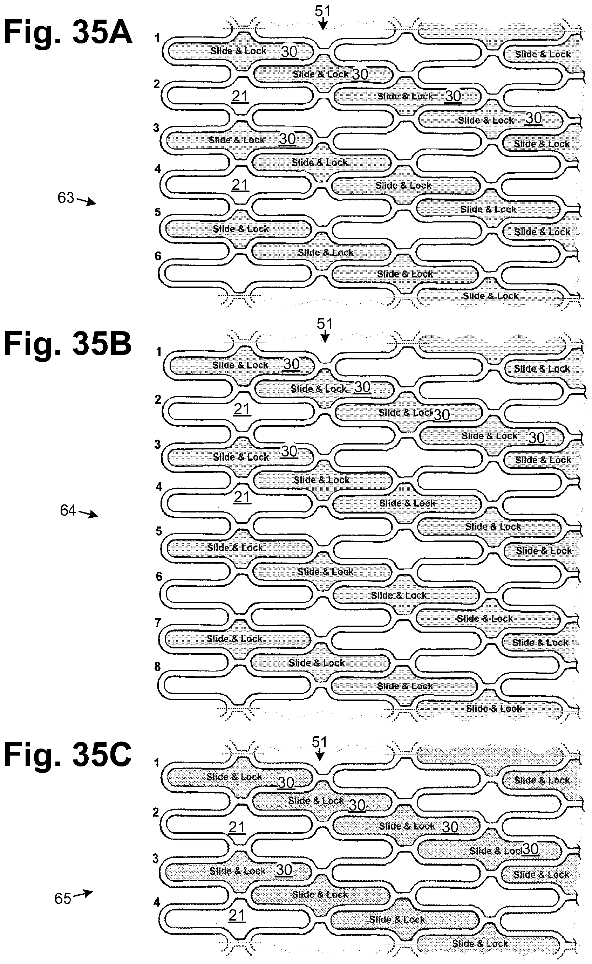

FIGS. 35A-C illustrate certain arrangements of the S&L braced cells in an embodiment having 1:2 spacing, having different patterns of adjacent S&L mechanisms and number of cells in each tubular ring.

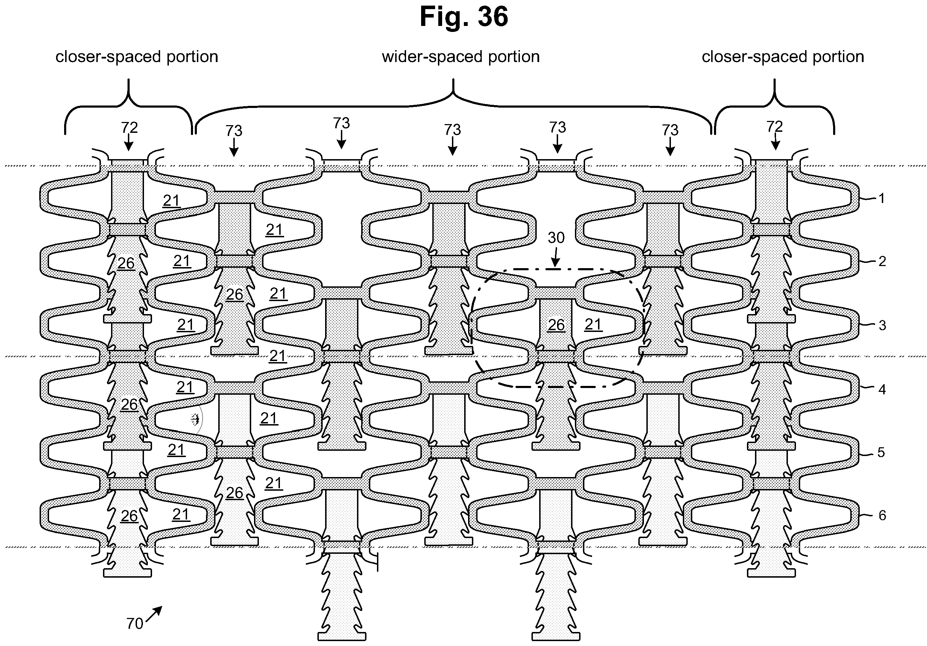

FIG. 36 illustrates an embodiment of a stent with rings of cells having different S&L spacing at different points along its longitudinal extent, such as having 1:2 spacing at proximal and distal end rings and 1:3 spacing for the rings between end-rings.

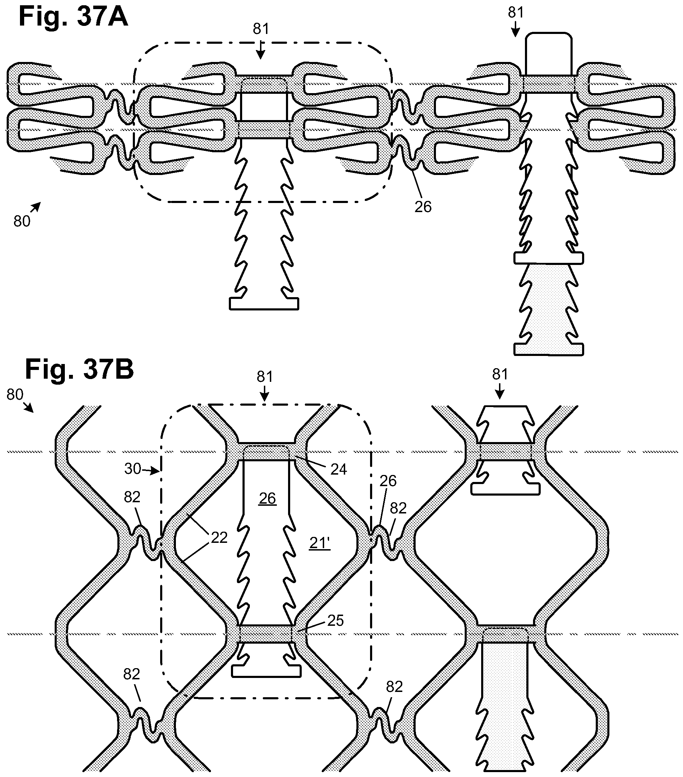

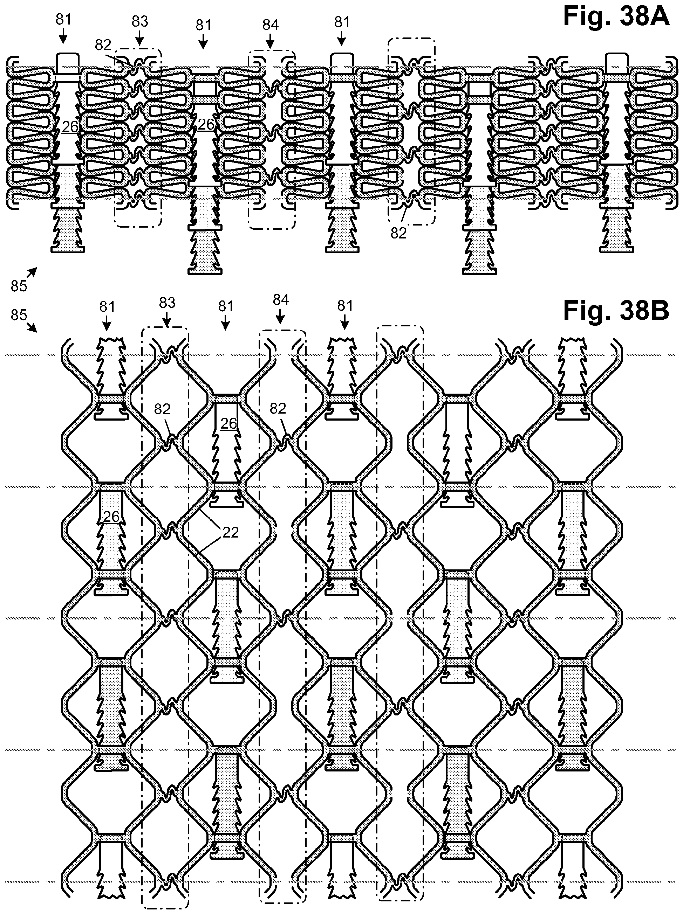

FIGS. 37A-B and 38A-B illustrate detailed and larger array views respectively of an embodiment in the compacted and expanded configurations, in which the rings of certain deformable cells are discretely separate from adjacent rings and/or have spaced apart interconnections between adjacent rings.

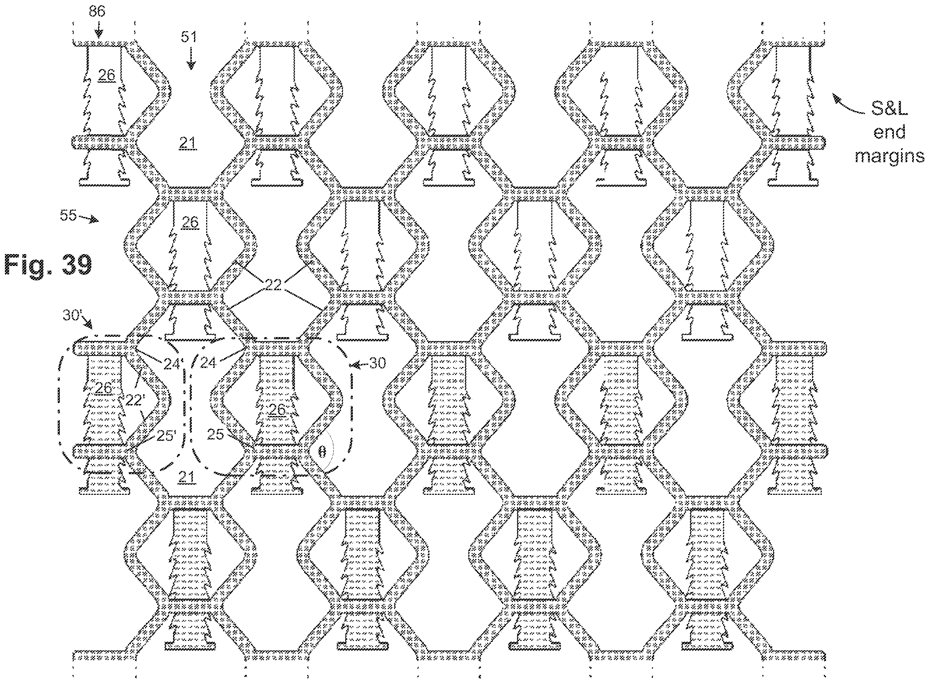

FIG. 39 illustrates an example of an embodiment of stent structure in which the proximal and distal ends of the stent are bounded by S&L mechanisms protruding longitudinally beyond the terminal cell ring.

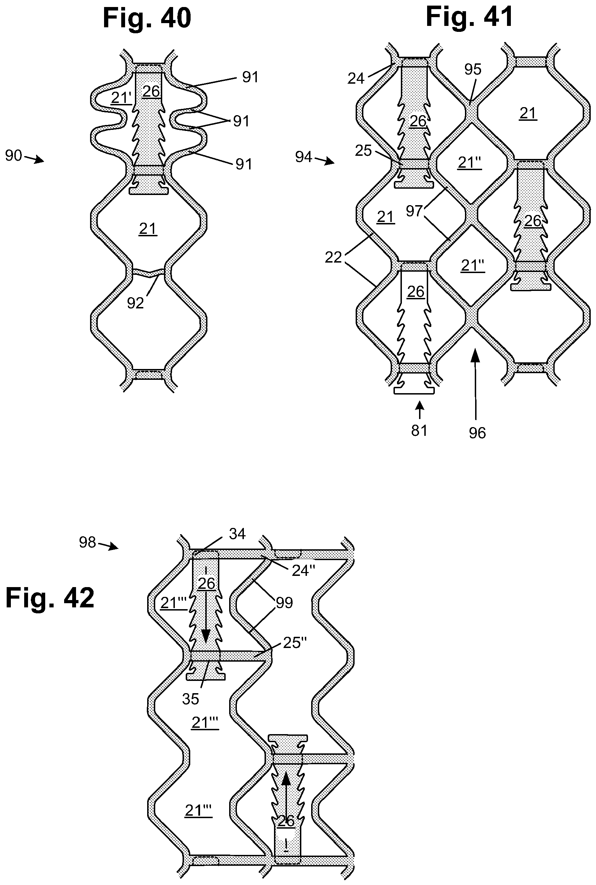

FIG. 40 illustrates an embodiment wherein one or more cells of a ring of cells are further subdivided by having multiple strut corners bordering the cell.
FIG. 41 illustrates an embodiment similar in some respects to the embodiment shown in FIGS. 37-38, in which intervening pairs of rings of struts (e.g., between cell rings having S&L mechanisms) are integrated or connected, so as to have common joint portions binding the adjacent strut rings.
FIG. 42 illustrates an embodiment having the rings of struts arranged in a peak-to-valley pattern (reference FIG. 20B) and the direction of the toothed rail members reverses in adjacent rings of cells.

### DETAILED DESCRIPTION OF CERTAIN EMBODIMENTS

The invention summarized above and defined by the enumerated claims may be better understood by referring to the following detailed description, which should be read in conjunction with the accompanying drawings. This description is provided to enable one having skill in the art to build and use the invention. It is not intended to limit the enumerated claims, but to serve as a particular example thereof.

While the description sets forth various embodiments in specific detail, it will be appreciated that the description is illustrative only and should not be construed in any way as limiting the same. Furthermore, various applications of the embodiments, and modifications thereto, which can occur to those who are skilled in the art, are also encompassed by the general concepts described herein. Any one feature, or any combination of features, of any of the embodiments can be combined with any of the other embodiments to yield additional embodiments within the scope of this disclosure.

This disclosure describes various embodiments of stent devices, systems, methods, and otherwise. The term "stent" is used herein to designate embodiments for placement in (1) vascular body lumens (i.e., arteries and/or veins) such as coronary vessels, neurovascular vessels and peripheral vessels for instance renal, iliac, femoral, popliteal, subclavian and carotid; and in (2) nonvascular body lumens such as those treated currently i.e., digestive lumens (e.g., gastrointestinal, duodenum and esophagus, biliary ducts), respiratory lumens (e.g., tracheal and bronchial), and urinary lumens (e.g., urethra); (3) additionally such embodiments can be useful in lumens of other body systems such as the reproductive, endocrine, hematopoietic and/or the integumentary, musculoskeletal/orthopedic and nervous systems (including auditory and ophthalmic applications); and, (4) finally, stent embodiments can be useful for expanding an obstructed lumen and for inducing an obstruction (e.g., as in the case of aneurysms).

The term "stent" is further used herein to designate embodiments such as; support structures for maintaining patency of a body lumen; support structures for anchoring thrombus filters and heart valves; as well as support structures for the distribution and delivery of therapeutic agents as well as other devices.

The term "stent" can be used interchangeably with the term "prosthesis" and should be interpreted broadly to include a wide variety of devices configured for supporting a segment of a body passageway. The term "body passageway" encompasses any lumen or duct within a body, such as those described herein.

Term "shape-memory material" is a broad term that can include a variety of known shape memory alloys, such as nickel-titanium alloys, as well as any other materials that return to a previously defined shape after undergoing substantial plastic deformation.

The term "radial strength," as used herein, describes the external pressure that a stent is able to withstand without incurring clinically significant damage.

Due to their high radial strength, balloon expandable stents are commonly used in the coronary arteries to ensure patency of the vessel. During deployment in a body lumen, the inflation of the balloon can be regulated for expanding the stent to a particular desired diameter. Accordingly, balloon expandable stents can be used in applications wherein precise placement and sizing are important. Balloon expandable stents can be used for direct stenting applications, where there is no pre-dilation of the vessel before stent deployment, or in prosthetic applications, following a pre-dilation procedure (e.g., balloon angioplasty). During direct stenting, the expansion of the inflatable balloon dilates the vessel while also expanding the stent.

The stent can be fabricated from one or more materials. These materials include metals, polymers and shape-memory materials. In another preferred embodiment, the stent further can comprise a tubular member formed from a biocompatible and preferably, bioresorbable polymer, such as those disclosed in US Publication No. 2006-0034769. It is also understood that the various polymer formulae employed can include homopolymers and heteropolymers, which can include stereoisomerism, composites, filled materials, etc. Homopolymer is used herein to designate a polymer comprised of all the same type of monomers. Heteropolymer is used herein to designate a polymer comprised of two or more different types of monomer which is also called a co-polymer. A heteropolymer or co-polymer can be of a kind known as block, random and alternating. Further with respect to the presentation of the various polymer formulae, products according to embodiments can be comprised of a homopolymer, heteropolymer and/or a blend of such polymers.

The term "bioresorbable" is used herein to designate polymers that undergo biodegradation (through the action of water and/or enzymes to be chemically degraded) and at least some of the degradation products can be eliminated and/or absorbed by the body. The term "radiopaque" is used herein to designate an object or material comprising the object visible by in vivo analysis techniques for imaging such as, but not limited to, methods such as x-ray radiography, fluoroscopy, other forms of radiation, MRI, electromagnetic energy, structural imaging (such as computed or computerized tomography), and functional imaging (such as ultrasonography). The term "inherently radiopaque" is used herein to designate polymer that is intrinsically radiopaque due to the covalent bonding of halogen species to the polymer. Accordingly, the term does encompass a polymer which is simply blended with a halogenated species or other radiopacifying agents such as metals and their complexes.

The stent further can comprise an amount of a therapeutic agent (for example, a pharmaceutical agent and/or a biologic agent) sufficient to exert a selected therapeutic effect. The term "pharmaceutical agent", as used herein, encompasses a substance intended for mitigation, treatment, or prevention of disease that stimulates a specific physiologic (metabolic) response. The term "biological agent", as used herein, encompasses any substance that possesses structural and/or functional activity in a biological system, including without limitation, organ, tissue or cell based derivatives, cells, viruses, vectors, nucleic acids (animal, plant, microbial, and viral) that can be natural and recombinant and synthetic in origin and of any sequence and size, antibodies, polynucleotides, oligonucleotides, cDNA's, oncogenes, proteins, peptides, amino acids, lipoproteins, glycoproteins, lipids, carbohydrates, polysaccharides, lipids, liposomes, or other cellular components or organelles for instance receptors and ligands. Further the term "biological agent", as used herein, can include virus, serum, toxin, antitoxin, vaccine, blood, blood component or derivative, allergenic product, or analogous product, or arsphenamine or its derivatives (or any trivalent organic arsenic compound) applicable to the prevention, treatment, or cure of diseases or injuries of man (per Section 351(a) of the Public Health Service Act (42 USC 262(a)).

The term "biological agent" can include 1) "biomolecule", as used herein, encompassing a biologically active peptide, protein, carbohydrate, vitamin, lipid, or nucleic acid produced by and purified from naturally occurring or recombinant organisms, tissues or cell lines or synthetic analogs of such molecules, including antibodies, growth factors, interleukins and interferons; 2) "genetic material" as used herein, encompassing nucleic acid (either deoxyribonucleic acid (DNA) or ribonucleic acid (RNA), genetic element, gene, factor, allele, operon, structural gene, regulator gene, operator gene, gene complement, genome, genetic code, codon, anticodon, messenger RNA (mRNA), transfer RNA (tRNA), ribosomal extrachromosomal genetic element, plasmagene, plasmid, transposon, gene mutation, gene sequence, exon, intron, and, 3) "processed biologics", as used herein, such as cells, tissues or organs that have undergone manipulation. The therapeutic agent can also include vitamin or mineral substances or other natural elements.

In some embodiments, the design features of the circumferentially offset elements can be varied to customize the functional features of strength, compliance, radius of curvature at deployment and expansion ratio. In some embodiments, the stent can comprise a resorbable material and vanishes when its job is done. In some embodiments, the stent serves as a therapeutic delivery platform.

Some aspects are also disclosed in US Patent Nos. 8,292,944; 7,473,417; 6,951,053; 8,236,340; and 7,763,065.

Some features and arrangements of embodiments of stents are disclosed in US Patent Nos. 6,033,436, 6,224,626, and 6,623,521, each issued to Steinke.

Advantageously, the stent design elements and interlocks can be varied to customize the functional features of strength, compliance, radius of curvature at deployment and expansion ratio. In some embodiments, the stent can comprise a resorbable material and vanishes when its job is done. In some embodiments, the stent serves as a delivery platform for therapeutic agents such as pharmaceutical compounds or biological materials.

Some embodiments relate to a radially expandable stent used to open, or to expand a targeted area in a body lumen. Some embodiments relate to a radially expandable stent used as a drug delivery platform to treat vascular conditions. In some embodiments, the assembled stent can comprise a tubular member having a length in the longitudinal axis and a diameter in the radial axis, of appropriate size to be inserted into the body lumen. The length and diameter of the tubular member can vary considerably for deployment in different selected target lumens depending on the number and configuration of the structural components, described below.

The tubular member in accordance with some embodiments can have a "clear through-lumen," which can be defined as having no structural elements protruding into the lumen in either the compacted or expanded diameters. Further, the tubular member can have smooth marginal edges to minimize the trauma of edge effects. The tubular member can be preferably thin-walled and flexible (e.g., less than about 0.01 Newtons force/millimeter deflection) to facilitate delivery to small vessels and through tortuous vasculature.

In some embodiments, the wall thickness can be about 0.0001 inches (0.00254 mm) to about 0.0250 inches (0.635 mm), and more preferably about 0.0010 to about 0.0100 inches (0.0254 to 0.254 mm). However, the wall thickness depends, at least in part, on the selected material. For example, the thickness can be less than about 0.0080 inches (0.2032 mm) for plastic and degradable materials and can be less than about 0.0020 inches (0.0508 mm) for metal materials. More particularly, for a 3.00 mm stent application, when a plastic material is used, the thickness can be preferably in the range of about 0.0020 inches (0.0508) to about 0.0100 inches (0.254 mm). The thin walled design can also minimize blood turbulence and thus risk of thrombosis. The thin profile of the deployed tubular member in accordance with some embodiments also facilitates more rapid endothelialization of the stent. The above thickness ranges have been found to provide preferred characteristics through all aspects of the device including assembly and deployment. However, it will be appreciated that the above thickness ranges should not be limiting with respect to the scope of the embodiments and that the present teachings can be applied to devices having dimensions not discussed herein.

The geometry of the stent may be generally described as a tubular member. In accordance with these various features, the slideably engaged expandable stent can include at least two slideably engaged radial elements defining a circumference of the tubular member.

The slidably engaged radial elements are configured for unidirectional slideable movement so as to permit the radial expansion of the tubular member. In a preferred embodiment, the stent will define a first compacted diameter, and a second expanded diameter. The slideably engaged expandable stent is adapted to be expandable between at least the first compacted diameter and at least the second expanded diameter.

In some embodiments, the slideably engaged expandable stent is configured with two radial modules, each radial module being slideably engaged and configured for unidirectional expansive movement. Each radial module has a backbone, a first elongate member and a second elongate member. In some embodiments, the elongate members are annular elongate members;

In some embodiments, the slidably engaged expandable stent is configured with two radial modules, each radial module being slidably engaged and configured for unidirectional expansive movement. Each radial module can include a backbone, a first elongate member and a second elongate member. In some embodiments, the elongate members are annular elongate members, such as ring-like members elongated from the backbone. The elongate members are slideably engaged with substantially captive slots and configured for unidirectional slideable movement.

Substantially captive slots are described as engagement slots which engage an elongate member with at least three sides, thereby holding the elongate member substantially captive and contained within the engagement means. Substantially captive slots in a preferred embodiment can be distributed along the backbone as well as at the distal end of the annular elongate members.

The slidably engaged expandable stent in a preferred embodiment has a plurality of annular elongate members, including a first elongate member and a second elongate member. These annular elongate members are substantially commonly oriented with respect to the backbone. Additionally, the second elongate member is circumferentially offset with respect to the first elongate member.

The circumferential offsetting of elongate members allows a distribution of slideable engagements. Such a distribution of slideable engagements is said to render the stent uniform with respect to mechanical failure points; as the slideable engagements are the weakest mechanical points in the design. Slideable engagements are herein defined as the engagement means between two slideably engaged radial modules. In a preferred embodiment, the slideable engagements are defined by the interlocking of substantially captive slots and contained rails of the slidably engaged elongate members.

The substantially captive slots can further comprise a locking member. A locking member can be a tooth, a deflectable tooth, or a stop. In a preferred embodiment, the substantially captive slots comprise a number of stops inside the surface or cavity of the slot. In another embodiment, the substantially captive slots comprise at least one tooth adjacent to the entry side of the substantially captive slot.

Additionally, the elongate members can be configured to comprise at least one conjugate locking member. A conjugate locking member is essentially a component designed to engage with the locking member. In a preferred embodiment, a conjugate locking member is adapted to fit be engaged by the locking member. In one embodiment, the conjugate locking member is one of a tooth, a deflectable tooth, or a stop. A locking member and a conjugate locking member define an engagement means whereby the radial modules are slidably engaged.

A conjugate locking member can be located on any part of the stent; however in a preferred embodiment, the conjugate locking member is located on the rail of an elongate member. Each elongate member has at least one radial surface and at least two axial sides. Axial sides are substantially perpendicular to the longitudinal axis of the elongate member. In a preferred embodiment; a plurality of conjugate locking members can be distributed on both axial sides of the rail. In one embodiment, the conjugate locking members on both axial sides of the elongate member can be substantially aligned in a mirrored distribution. In another embodiment, the conjugate locking members can be substantially mirrored but offset by a vertical distance with respect to the opposite axial side. Axial locking members can be axially nested and substantially prevented from protruding into the vessel wall, thereby preventing undesired agitation which can cause stenosis.

Further, conjugate locking members can be spaced apart by a defined distance. The conjugate locking members on one axial side can be offset with respect to the conjugate locking members of the second axial side. Such an offsetting of conjugate locking members can provide a higher resolution for stent diameter customization.

In another embodiment, the stent comprises a backbone adapted to substantially coil about the tubular member. A substantially coiled backbone; or otherwise herein referred to as a helical backbone or a flexible backbone, gives rotational flexibility to the stent design. Rotational flexibility can be an important improvement which will allow the stent to adapt to vasodynamic movements. A substantially coiled backbone can be an elongate backbone configured to coil about the tubular member, or alternatively can be a stair-step pattern, a wave-like pattern, or any other pattern which is substantially configured in a helical orientation about the tubular member.

In one embodiment, a plurality of radial elements each comprising a backbone can be configured into a tubular member having a plurality of substantially coiled backbones. A flexible backbone is herein defined as any backbone of a radial element which is configured to substantially coil about the tubular member.

Additionally, a flexible backbone can comprise a flexible link in the backbone, such as a spring link. Or alternatively, the flexible backbone can be made of an elastomeric polymer material sufficient to promote adaption to vasodynamic movements. Elastomeric polymers are defined in the art, however for illustrative purposes examples can include polycaprolactone, polydioxanone, and polyhexamethylcarbonate.

### EXAMPLES

FIGS. 1-5 illustrate aspects of an embodiment of a tubular slide & lock stent assembly 100. Since some features and functions are more clearly indicated in various figures, FIGS. 1-5 should be understood in combination.

FIG. 1 is a planar representation of an embodiment of a tubular slide & lock stent assembly 100 (also called a stent or a stent structure) in a compacted configuration. In various embodiments, the lateral dimension represents the longitudinal axis of the stent and the vertical dimension represents the circumferential direction of the tubular assembly. The planar surface representing the outside of the tubular shape, and the upper portion of the figure may be conceived as wrapping around into the page to join the bottom portion of the figure.

FIG. 2 is a planar representation of a tubular slide & lock stent assembly 100 of FIG.1, shown in a partially expanded configuration.

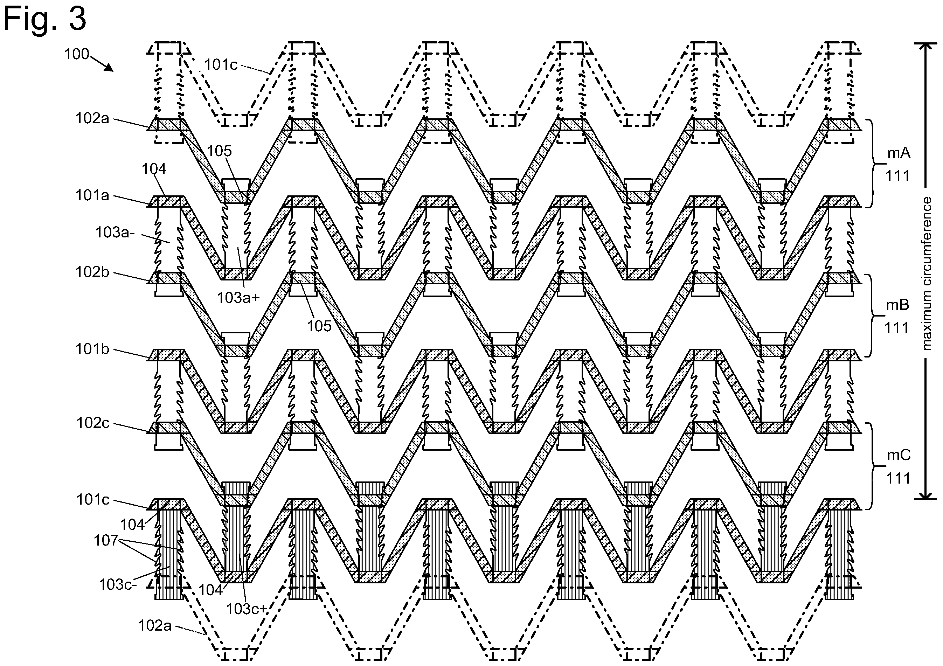

FIG. 3 is a planar representation of a tubular slide & lock stent assembly 100 of FIG.1, shown in a fully expanded configuration. This expanded depiction may be more clearly illustrative of some features.

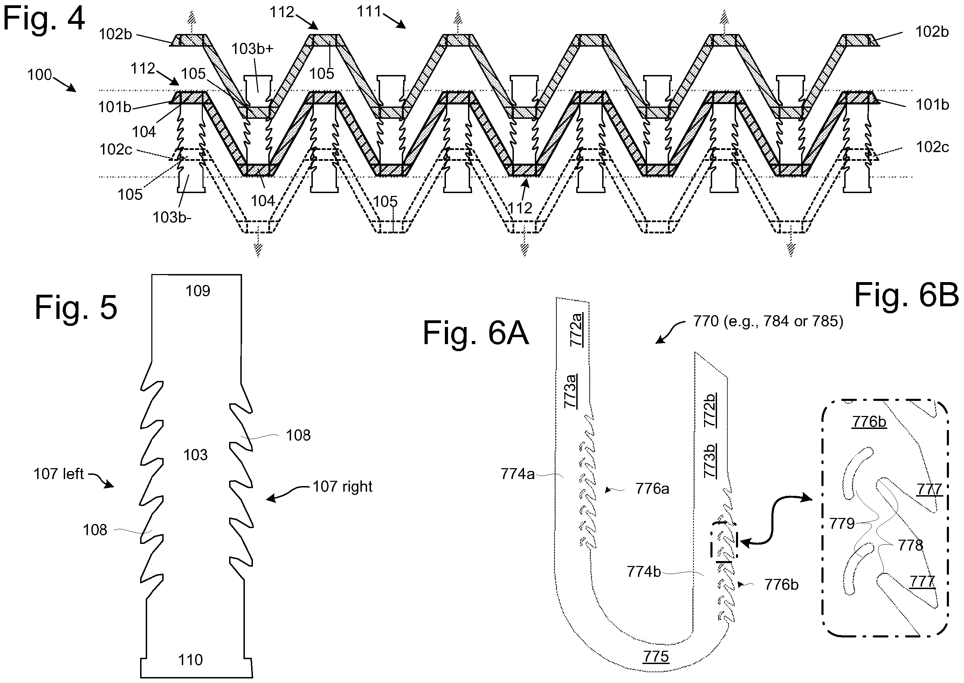

FIG. 4 is a planar representation of a portion of the assembly of FIGS. 1-3, in a partially expanded configuration similar to FIG. 2. As shown, the stent can include a longitudinal module comprising a pair of backbones with an associated rail array.

FIG. 5 depicts an embodiment of a single rail member as included in stent 100, and certain details of its structure.

The stent 100 comprises a plurality of circumferentially-repeating longitudinal modules 111. The modules 111 can comprise a longitudinally-extending bonding backbone 101 (also called a "bond backbone"). In some embodiments, the modules 111 include a clockwise (or downwardly-directed in FIG. 4) array of longitudinally spaced-apart rail members 103- and a counter-clockwise (or upwardly-directed in FIG. 4) array of longitudinally spaced-apart rail members 103+. In some implementations, such as is shown in FIGS. 3 and 4, the circumferential direction of the rail members alternates along the longitudinal axis. For example, a first rail member can extend in a clockwise direction, a second rail member that is longitudinally adjacent to the first rail member is can extend in a counter-clockwise direction, and a third rail member that is longitudinally adjacent the second rail member is can extend in the clockwise direction. Each of the rail members 103-and 103+ can be bonded and/or fixed within a corresponding bond channel 104. As illustrated, the slots 104 can be arranged longitudinally along the backbone 101.

Note that the use of the "+" and "-" symbols in indicating portions of the stent is for nomenclature purposes only and does not designate, e.g., an electrical charge or polarity of the stent components. Also, the use of the terms "bonding backbone" and "bond backbone" does not necessarily mean that rail member 103 need be adhesively bonded to backbone 101. Although various bonding compositions and adhesive materials may be advantageously employed to mount rail 103 fixedly to backbone 101, other connection methods may be used alternatively or in combination. For example, the rail member 103 can be connected with the backbone 101 by welding (such as ultrasonic welding), pin structures, monolithic structures, pivoted structures, or otherwise.

As shown, the module 111 can include an engagement-slot backbone 102 (also called a "slot backbone"). The engagement-slot backbone 102 can be arranged generally parallel and circumferentially adjacent to the bond backbone 101. In the example shown, the slot backbone 102 is shown above (counter-clockwise) the backbone 101, but other embodiments have different configurations. In some implementations, the engagement-slot backbone 102 has a shape that is complementary to the shape of the backbone 101. For example, as shown in FIG. 1, the backbones 101, 102 can nest together. This can reduce the size of the stent 100.

The slot backbone 102 can have a first array of rail/tooth engagement slots 105 arranged longitudinally along backbone 102. The engagement slots 105 can be positioned or otherwise configured to engage a corresponding one of the upwardly-directed rail members 103+. The slot backbone 102 can have a second array of rail/tooth engagement slots 105 arranged longitudinally along backbone 102, each positioned or otherwise configured to engage a corresponding one of the spaced-apart rail members of an adjacent module, as shown in the planar array of three modules in FIGS. 1-3. In certain implementations, the first array of rail/tooth engagement slots 105 is circumferentially offset from the second array of rail/tooth engagement slots 105. For example, as shown, the first array of rail/tooth engagement slots 105 can be located at a peak of the slot backbone 102 and the second array of rail/tooth engagement slots 105 can be located at a valley of the slot backbone 102. As shown in dashed lines in FIG. 4, slots 105 of a slot backbone 102c of an adjacent module can engage the rail members 103b- and/or slots 105 of a slot backbone 102b of an adjacent module can engage the rail members 103b+.

As shown, the backbones can include a plurality of struts 122. In some implementations, the struts 122 are substantially linear segments. When the stent 100 is in the compacted state and/or when the stent is in the expanded state, the struts 122 are not oriented parallel with the longitudinal axis. In certain embodiments, one or more of the struts 122 can extend at an angle relative to the longitudinal axis, such as greater than or equal to about: 30°, 45°, 50°, 65°, values between the aforementioned values, or otherwise. In some variants, each bond channel 104 connects with at least one of the struts 122 and/or each engagement slot 105 connects with at least one of the struts 122.

As shown in FIGS. 1-3, the tubular stent assembly 100 includes three modules 111, designated mA, mB and mC. In some embodiments, each of mA, mB, and mC comprise a bond backbone 101 and a slot backbone 102. Alternative embodiments need not have three modules 111, but may have more or fewer. For example, some alternative embodiments (not shown) may have two modules, or four modules. The modular concept may be employed to make stents of different sizes (diameters or lengths), or different deployment characteristics, as suited to an application.

As illustrated, as the tubular stent assembly 100 is progressively expanded from a compacted form of FIG. 1 ("minimum circumference") to a fully expanded form of FIG. 3 ("maximum circumference"), it can be seen that each module expands in a similar fashion. In various embodiments, the expansion of the stent need not be longitudinally or circumferentially uniform, either during deployment or in final expanded form. Rather, the expansion during and following deployment from a balloon catheter may vary considerably, e.g., due to particular vascular geometry, lesion conformation and hardness, and the like. Likewise, the stent 100 need not necessarily maintain a circular tubular cross-section, either during deployment or in the final deployed form, due to the same factors.

As illustrated in FIG.4, in the embodiment 100, the backbones 101, 102 are shown as having an overall "Zig-Zag" form. For example, each slot portion 104-105 can be generally parallel to the longitudinal axis, and each intermediate portion can be formed at a substantial angle to this axis while remaining aligned to the tubular shape. In some implementations, the backbones 101, 102 are generally sinusoidally shaped, undulating, or otherwise. As illustrated, the backbones 101, 102 can include a longitudinal series of shapes, such as generally trapezoidal shapes (with an open bottom of the trapezoid), generally triangular shapes (with an open bottom of the triangle), or otherwise. As shown, the backbones 101, 102 can include a series of alternatingly-oriented shapes (e.g., each shape is turned about 180° relative to the longitudinally adjacent shapes).

In some implementations, the backbones 101, 102 include reversing portions 112 at which the slope of the backbone 101, 102 are relative to the longitudinal axis changes from positive to negative or negative to positive. In some such variants, the reversing portion 112 is substantially linear. For example, the reversing portion 112 can extend generally parallel with the longitudinal axis. In some variants, the reversing portion 112 is curved.

The congruent shape of the bonding backbones 101 and slot backbones 102 permits advantageous nesting in the compact configuration. The parallel direction of the slotted portions can promote smooth sliding of rails 103 in slots 105. Also, the angled intermediate portions and over-all "Zig-Zag" form can promote lateral flexibility of the tubular stent assembly. This can be advantageous, such as in both vascular insertion and in conforming to vascular geometry. However, in other embodiments of the stent 100, both these factors may be different. For example, the slots need not be parallel to the longitudinal axis (e.g., may lie at angles (see, e.g., aspects of FIGS. 8A-B) and/or the backbones may be curved, generally sinusoidal, generally helical, or even straight.

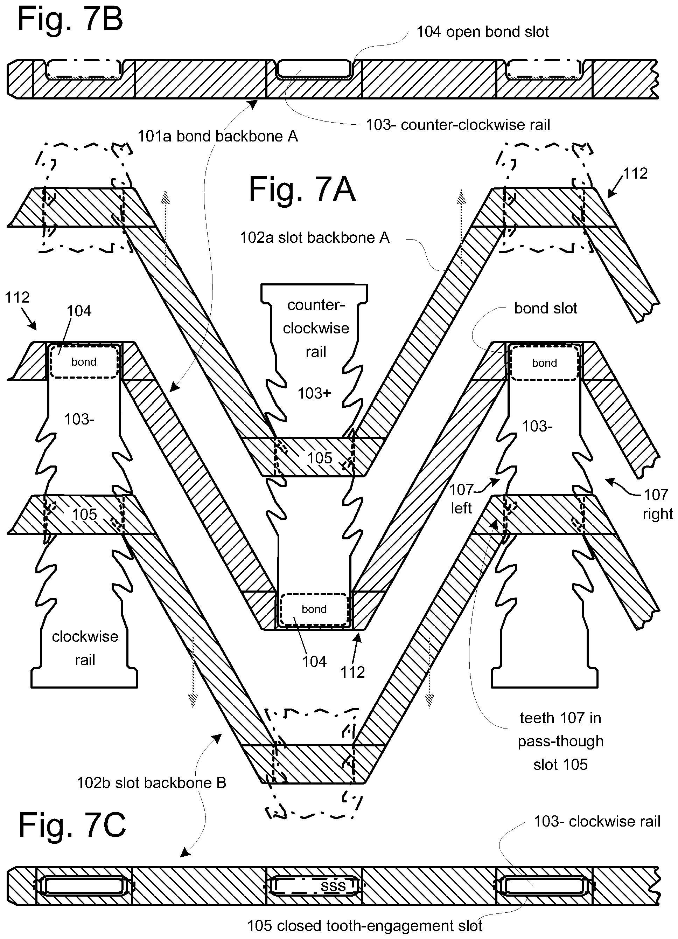

FIGS. 7A, 7B, and 7C illustrate details of the stent assembly embodiment 100 shown in FIGS. 1-3. FIG. 7A is a detail of a portion of the planar array of FIG. 2. FIG. 7B is a cross-section of a portion of a bond backbone 101 showing details of the bond channels 104. FIG. 7C is a cross-section of a portion of an engagement/locking backbone 102 showing details of the pass-through or tooth engagement slots 105. As illustrated, each of rail members 103 can have one or more arrays 107 of locking teeth 108. For example, the rail members 103 can have a pair of arrays 107 (indicated a 107 left and 107 right arbitrarily), such as one on each side of rail 103. In some embodiments, each of rails 103 is arranged to pass through a slot 105 of a corresponding slot backbone 102, thereby slidably engaging the slot 105. In certain implementations, the teeth 108 engage with walls of the slot 105, which can allow sliding in one direction only. For example, the teeth 108 can be configured to "lock" or resist recoil of the backbones 101 and 102 of module 111. This can allow expansion of stent 100 without permitting subsequent compaction (see arrows in FIG. 7B). As shown in FIG. 5, the rail 103 can include a proximal end 109 configured to engage and be fixed to bond channel 105. A distal end of the rail 103 includes a stop portion 110 to stop sliding motion or rail 103 in slot 105, so as to prevent overexpansion of the stent 100.

For purposes of comparison and for illustrating certain features, FIGS. 6A and 6B are substantially Figures 11A and 11B, respectively, of US Patent No. 8,523,936 (Application No. 13/083,508 filed April 8, 2011). These figures illustrate a "U" shaped rail member of an embodiment of a stent of the '508 application.

FIGS. 6A and 6B show the action of engagement teeth or locking members provided on the elongate elements or rails of the rail members, configured for engagement with slots in the backbones of the stent embodiments described herein. In the example of FIGS. 6A and 6B, the rail member 770 includes a pair of elongate rails 773a and 773b. Of course it should be understood that various embodiments of the rail member 770 can include more than two elongate rails. For example, some implementations of the rail member 770 have three, four, five, six, or more elongate rails.

A reference frame for the rail member 770 may be defined relative to the backbone to which it is to be fixedly mounted, joined or bonded in the assembled stent. In this reference frame, each of rails 773a-b of member 770 has a proximal end 772a-b configured to be fixedly mounted or bonded to a supporting backbone. In this reference frame, the rail elements 773a-b, as assembled, will extend distally to engage a sliding slot 787 of an adjacent backbone. Each of rails 773a-b includes a medial portion 774a-b supporting a locking mechanism 776a-b. In the illustrated embodiment, the locking mechanism 776 is disposed on only one side of rail 773. Other embodiments have at least some of the locking mechanism 776 disposed on both sides of the rail 773a and/or 773b. The rails 773a and 773b are joined at their distal ends by cross-member 775. In alternative embodiments, where member 770 has more than two rails, cross-member 775 may join more than two rails.

In the detail drawing of FIG. 6B, it may be seen that the locking mechanism 776 comprises a sequential plurality of locking elements 777, which are illustrated as being tooth-like in this example. The locking elements 777 can be separated by indented connecting regions 778. In some cases, at least one stress management or relieving opening 779 is disposed adjacent connecting regions 778. The relieving opening 779 can facilitate adjustment of the characteristics (e.g., flexibility, rebound, and the like) of the locking elements 777 by the selection of the shape, position, and/or size of the opening 779.

A comparison of FIG. 5 with FIGS. 6A-B shows that the single rail form of the rail member 103 of FIG. 5 provides symmetry of form, with balanced forces left-to-right from tooth engagement. This can be advantageous over the asymmetrical form of the rail member 770 in FIGS. 6A and 6B. In some embodiments, the backbones 101, 102 can be free to flex longitudinally without less effect on the interaction with the rail member 103 (e.g., since only one pair of slots 104,105 engage the rail 103) compared to the rail member 770 of FIGS. 6A and 6B.

For purposes of comparison and for illustrating certain features, FIGS. 8A and 8B are substantially Figures 12A and 12B, respectively, of US Patent No. 8,523,936 (Application No. 13/083,508 filed April 8, 2011). These figures illustrate an alternative tubular stent assembly having rail modules with rail members in planar representation in compacted and expanded configurations, respectively.

For purposes of comparison, FIGS. 8A and 8B depict a stent embodiment 780 in which additional modules of rail members are included. The embodiment 780 has at least a first module of rail members 784 directed in a generally perpendicular direction relative to the stent axis (e.g., downward in the illustrations of FIGS. 8A and 8B), and at least a second module of rail members 785 directed generally opposite the first module of rail members 784 and generally perpendicular relative to the stent axis (e.g., upward in the illustrations of FIGS. 8A and 8B).

Each of the modules 784, 785 can comprise a group of three overlapping rail members 784a-c, 785a-c. Each rail member can be joined or mounted to at least one backbone (three backbones 782a-c are illustrated) at attachment point 786. Each rail member 784 can comprise a plurality of space-apart generally parallel rails 784', 784" that can be connected by a distal cross member 784"'. Rail members 785 can be similarly structured.

In addition to being proximally mounted to at least one backbone, each of the individual rails or the rail members 784a-c, 785a-c can engage and pass slidably through a slot 787 in an adjacent backbone. As discussed above, although FIGS. 8A and 8B diagrammatically show assembly 780 as planar shape, these figures represent a generally tubular stent assembly 780. As shown by arrow 788a, the rail members 784c mounted to backbone 782c can be configured to pass slidably through slots of adjacent backbone 782a, and similarly arrow 788b indicates that rails member 785a mounted to backbone 782a can be configured to pass slidably through slots in backbone 782c.

FIG. 8A illustrates a compacted form of stent assembly 780, in which the toothed portion 789 of each rail of rail members 784a-c, 785a-c is positioned distal to the corresponding engagement slot 787 (indicated as 787a). In some embodiments, the backbones 782a-c are disposed closely adjacent one another and/or nested. In some embodiments, in the compacted configuration, the stent 780 is configured to mount on a compacted balloon catheter.

FIG. 8B illustrates a radially expanded form of the stent assembly 780, in which the toothed portion 789 of each rail of the rail members 784a-c, 785a-c is positioned at least partially within the corresponding engagement slot 787 (indicated as 787b). In some embodiments, in the radially expanded state of the stent 780, the backbones 782a-c are disposed at a substantial separation from one another, as would typically be configured for deployment at a larger tubular diameter supporting a treated vascular or other body lumen. In this configuration, the toothed portion 789 can inhibit radial contraction of the stent 780.

FIGS. 9A-9C illustrate the stent 100 of FIGS. 1-3, respectively. In FIGS. 9A-C, the tubular stent assembly 100 is depicted end-on, from a perspective view along the stent longitudinal axis. In FIG. 9A, the stent 100 is shown in compact configuration, as it might be when mounted on a balloon catheter 120 in a clinical product. In FIG. 9B, the stent 100 is shown partially expanded, the balloon 120 inflating as shown in the diverging arrows, and the backbones moving to greater spacing as shown by the counter-clockwise arrows. In FIG. 9C, the stent 100 is depicted as generally fully expanded or deployed. As noted above, the stent need not deploy as a circular section tube, but may be elliptical or irregular and the like, e.g., due to particular vascular geometry, lesion conformation and hardness, and the like.

FIGS. 10A and 10B illustrate the stent 100, in approximately isometric perspective views, in compacted and expanded configurations. As shown, the stent 100 can go from compact to a tightly compact form. This can be facilitated by tight nesting of the backbones 101-102 and/or by overlapping of the rails 103 in adjacent modules. As shown in FIG. 10A, when the stent is in the fully compacted state (e.g., on a deflated balloon), circumferential surfaces of each of the backbones 101 contact corresponding circumferential surfaces on circumferentially adjacent backbones 102 and/or circumferential surfaces of each of the backbones 102 contact corresponding circumferential surfaces on circumferentially adjacent backbones 101.

FIGS. 11A, 11B, 14A, and 14B show examples of dimensions (in inches (mm in parenthesis)) and angles (in degrees) of the backbones 101-102 and rails 103 described above. Of course, the indicated dimensions and angles are merely illustrative; in other embodiments, the dimensions and/or angles may be different (e.g., substantially greater or substantially less) than shown. As discussed above, there are many alternative shapes of the backbones 101 or 102 that may be employed without departing from the spirit of the invention. Likewise the shape and pitch of teeth 108 may vary substantially from what is shown.

FIG. 12 shows certain configurations of the bond channel 104. As shown, in some embodiments, the bond channel 104 is open on one or more sides. For example, as shown, the slot 104 can be open in a radial direction, which can allow the rails 103 to be radially received into the slot 104. In some embodiments, the bond channel 104 is closed (indicated as slot 104x). For example, the bond channel 104x can be enclosed by the backbone structure. In some such embodiments, the rail 103 cannot be radially received into the slot 104x. In various implementations, the rail 103 can slidingly move in the slot 104, 104x in a circumferential direction to facilitate expansion of the stent 100.

FIGS. 13A and 13B illustrate certain embodiments of the tubular stent 100 in the contracted state and the expanded state. As in several other of the illustrations herein, for purposes of presentation, the tubular stent is shown in a planar representation. As shown, the bond backbone 101 and/or the slot backbone 102 can include one or more link elements 182. The link elements 182 can provide longitudinal and/or torsional flexibility, which can allow the stent 100 to elongate and/or twist. This can facilitate positioning of the stent 100 in a tortuous and/or irregularly shaped body lumen. As shown, in certain embodiments, the link elements 182 are positioned in certain of the struts that form angled portions (e.g., relative to the longitudinal axis) of the backbones. In some embodiments, the link elements 182 are curved, undulating, and/or zig-zagged portions. In certain variants, the link elements 182 are generally S-shaped or generally U-shaped portions. Circumferentially adjacent link elements 182 can have complementary shapes, which can facilitate nesting of the link elements 182 when the stent 100 is in the compacted state.

FIGS. 14C-F illustrate top and side views of certain configurations of the backbones 101, 102. For example, as shown in FIGS. 14C and 14E, the backbone 101 can include open slots 104. In other embodiments, the slots 104 are closed. The slots 104 can have a radial thickness that a portion of the overall thickness of the backbone 101, such as less than or equal to about: 2/3, 1/2, 1/3, 1/4, 1/8, values between the aforementioned values, or otherwise. As shown in FIGS. 14D and 14F, the backbone 102 can include closed slots 105. In some embodiments, the slots 105 are open. In some variants, the backbone 102 has a generally constant radial thickness. In certain implementations, the backbone 102 has a variable radial thickness. For example, the backbone (indicated as 102') can have a recessed radial profile between the slots 105. For example, as a percentage of the non-recessed radial thickness of the backbone 102, the radial thickness of the recessed portions can be at least about: 50%, 60%, 75%, 80%, 90%, 95%, values between the aforementioned values, or otherwise. The slots 104, 105 and/or the backbones 101, 102 can have any of the aforementioned features (e.g., the slot 104 can have any of the aforementioned features of the slot 105 and vice versa, and the backbone 101 can have any of the aforementioned features of the backbone 102 and vice versa).

### Stent structures with plastically deformable elements

For context, FIGS. 15, 16, 17A-17D, 18, and 19 reproduce FIGS. 13, 4, 7-10, 11, and 5, respectively, from US Patent No. 5,514,154. The stents in these figures are representative of certain known stents having portions allowing the stent to be expanded from a compacted configuration (e.g., via a balloon catheter) to a deployed configuration.

The stent of FIG. 15 includes six peaks labeled 1' through 6' (not shown in the patent, but added to identify the peaks). The stents of FIGS. 15 and 16 have circumferential rings formed of struts (e.g., No. 31 in FIG. 15 and No. 12 in FIG. 16) connecting at each end to two adjacent struts, and arranged in an overall undulating or zig-zag configuration. The stents of FIGS. 15 and 16 also have a number of interconnecting linking elements 13 between longitudinally adjacent rings of struts. In the examples shown, there are three such interlinks between each ring of struts.

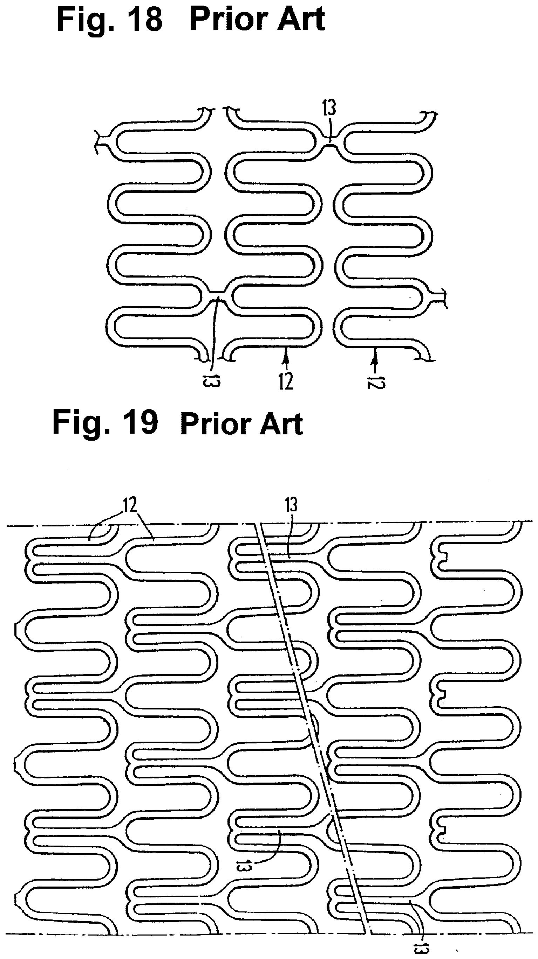

FIGS. 17A-D schematically show particular ways that rings of struts or other tubular ring-shaped stent elements (such as multi-strut cells) may be interconnected. The stents of FIGS. 18 and 19 show examples of tubular arrays of ring structures, suitable for expandable stent embodiments.

FIGS. 20A and 20B show certain tubular arrays of the ring structure that are, in some respects, similar to those shown in FIGS. 18 and 19. These two examples may be described as a Peak-to-Peak arrangement (FIG. 20A) and a Peak-to-Valley arrangement (FIG. 20B).

FIGS. 21A-C illustrate the compacted to expanded configurations of a portion of a stent 10. The stent can include a plurality of deformable components, such as struts 12. In some embodiments, during radial expansion of the stent, the deformable components undergo plastic deformation and/or strain hardening. This can provide resistance to recoil, that is, resistance to a tendency to re-compact or reduce internal tubular diameter under vascular stress within the body of a patient. The struts 12 can be connected to one another to form circumferential rings (e.g., FIGS. 21A-C may each illustrate portions of two of the rings). As shown, the rings can have a wavy, zig-zag, substantially sinusoidal, or other generally undulating shape. In various embodiments, each of the rings (also called annular supports) has a series of peaks and valleys.

In some embodiments, the adjacent such rings are cross-linked by link 13 to form a sequence of cells 14 in circumferential arrangement. The cells can be defined by the struts 12 of the rings and by the links 13. For example, the embodiment of FIGS. 21A-C comprises a first cell (defined by four struts 12 and two links 13) and second and third cells (each defined by four struts 12 and one link 13).

FIGS. 22A-D illustrate the compacted to expanded configurations of a stent sub-structure 20 comprising a circumferential ring of cells similar to those shown in FIGS. 21A-21C. As shown, certain of the cells can be connected to a slide and lock (S&L) mechanism 30 to resist recoil in the expanded state.

FIGS. 22A-C illustrate an example embodiment of a tubular stent 20. For purposes of presentation, the stent 20 is shown in a planar view, though the stent is tubular in shape. The structure illustrated may represent some or all of a stent. The general layout of the stent 20 in this particular example is, in some respects, similar to that of structure 10 in FIGS. 21A-C. As shown, the stent 20 can include comprising one or more circumferential rings of cells 21. The cells can be partially defined (e.g., longitudinally) by one or more (e.g., two) undulating annular supports. In various embodiments, the undulating annular supports are connected with a plurality of cross members. The cross members can partially define the cells as well (e.g., circumferentially). In the embodiment illustrated, the stent 20 has 3 cells 21 in circumferential sequence, and may form a portion of a tubular ring having, for example, a total of 6 cells per tubular ring. Other embodiments have a different number (e.g., 2, 4, 5, 6, 7, 8, or otherwise) of cells in circumferential sequence and/or form a portion of a tubular ring having different number of cells (e.g., 4, 5, 7, 8, 9, 10, 11, 12, or otherwise) per tubular ring.

As illustrated, the undulating annular support can include a plurality of struts 22. The struts 22 can be configured to deform, which can facilitate expansion of the stent 20. For example, the struts 22 can bend, twist, curve, reverse, swing or otherwise. Such deformability can allow the struts 22 to rotate relative to the longitudinal axis during expansion of the stent 20. For example, from the fully compacted state to the fully expanded state, some or all of the struts 22 can rotate relative to the longitudinal axis by at least approximately: 60°, 70°, 80°, 90°, 100°, 110°, 120°, values between the aforementioned values, or otherwise. Rotation of the struts 22 can facilitate radial expansion of the circumferential rings formed by the struts, thereby allowing the stent 20 to radially expand.

As shown in Figures 22A and 22C, the struts 22 can be substantially linear segments. When the stent is in the compacted state and/or when the stent is in the expanded state, the struts 22 are not oriented parallel with the longitudinal axis. In certain embodiments, one or more of the struts 22 can extend at an angle (e.g., less than or equal to about: 5°, 10°, 20°, 30°, values between the aforementioned values, or otherwise) relative to and on a first side of the longitudinal axis in the compacted state. In some embodiments, the one or more of the struts 22 can extend at an angle (e.g., greater than or equal to about: 30°, 45°, 60°, values between the aforementioned values, or otherwise) relative to and on a second side of the longitudinal axis in the expanded state.

During the course of radial expansion of the stent 20, the stent is in an intermediate state, which is a state between the compacted state and the expanded state. In certain variants, some, substantially all, or all of the struts 22 are substantially parallel with the longitudinal axis when the stent 20 is in the intermediate state.

In certain embodiments, the stent 20 includes first and second undulating annular supports. In certain embodiments, the struts 22 form the undulating annular supports. As shown, the first and second undulating annular supports can be arranged in a Peak-to-Peak arrangement (see FIG. 20A). For example, as shown in FIGS. 22A-C, the first undulating annular support can be about 180 degrees out of phase with the second undulating annular support. This can result in the peaks of the first and second undulating annular supports being generally circumferentially aligned and/or the valleys of the first and second undulating annular supports being generally circumferentially aligned.

As noted above, in some embodiments, the stent 20 comprises a slide and lock (S&L) mechanism 30. The S&L mechanism 30 can be similar or identical to (and can include any or all of the details and functional aspects of) the slide and lock mechanisms described above, such as with respect to the embodiments of FIGS. 1-14. For example, the S&L mechanism 30 can allow radial expansion of the stent 20 and inhibit or prevent radial contraction of the stent 20.

FIG. 22E is a multi-view illustration of certain details of the illustrative S&L mechanism 30 of FIGS. 22A-D. The S&L mechanism 30 can reside in and/or encompass one of cells 21 (e.g., see the dashed line in FIG. 22C). The S&L mechanism 30 can include a first cross member 24 and a second cross member 25. The first cross member 24 can include a mounting slot 34 (e.g., an open or closed slot) for mounting a proximal end 31 of a toothed rail 26, such as by adhesive bonding, sonic or thermal welding, or otherwise. The second member 25 can include a pass-through slot 35 (e.g., an open or closed slot) through which an intermediate portion 33 of the rail 26 passes. In some embodiments, the intermediate portion 33 includes the midpoint of the circumferential length of the rail 26. The rail 26 can include a ratcheting mechanism (e.g., teeth 38), which can resiliently deflect to permit one-way passage of the rail portion 33 through the slot 35. The teeth 38 can spring-back to lock the rail 26 so as to prevent re-compaction or recoil of the stent 20.

During expansion of the stent 20, an end stop 32 formed at the distal end of the rail 26 limits the travel of the rail 26 relative to the second member 25. This can limit the maximum amount of expansion of the stent 20 and/or can inhibit or prevent over-expansion expansion of the rail 26. In certain embodiments, the end stop 32 is configured to avoid disengagement of the rail 26 from slot 35 (e.g., by providing an interference with the slot 35).

In stent 20 of FIGS. 22A-C, there is one S&L mechanism 30 included in a sequence of three cells 21. This is referred-to herein as a S&L spacing of 1:3. FIG. 22D shows an illustrative alternative structure 40 in which there is one S&L mechanism 30 included in a sequence of two cells 21, thus having a S&L spacing of 1:2. Some embodiments include a S&L spacing of 1:1. Certain embodiments have other S&L spacings, such as 1:4, 1:5, 1:6, 1:7, or otherwise. Some variants include a combination of S&L spacings, such as 1:2 and 1:3, 1:3 and 1:4, 1:5 and 1:1, and combinations thereof.

FIG. 23 illustrates an embodiment of a tubular array stent structure 50 represented in planar schematic. As shown, the stent structure 50 can include circumferential rings 51 of cells having spaced-apart S&L mechanisms 30 bracing certain ones of the cells (S&L braced cells). In some embodiments, adjacent rings of cells (51, 51') are integrated so that each ring (except end rings) forms part of two adjacent rings of cells 21.

In the example of FIG. 23, each ring 51 can comprise two units of the stent 20 of FIGS. 22A-C, connected end-to-end. In this example, each ring of struts 22 comprises 12 struts 22. Each ring 51 of cells 21 comprises 6 cells 21. The S&L mechanisms 30 are spaced 1:3 (one mechanism 30 per 3 cells 21). The 9 rings 51/51' of array 50 are arranged in a staggered pattern so that each mechanism 30 of ring 51 is circumferentially offset from each corresponding mechanism 30 of the adjacent ring 51'. This is merely a representative example of the many possible alternative tubular stent array structures within the scope of this disclosure, as may be seen from the further representative examples described elsewhere herein.

FIGS. 24A-C illustrate compacted (24A), intermediate (24B), and expanded (24C) configurations of an embodiment of a stent structure 50 having a plurality of struts 22 and being generally similar to FIG. 23. Such an embodiment is illustrative of the large range of expansion provided by certain embodiments of the deflectable and slide and lock stent disclosed herein. As illustrated, the stent can have a longitudinal length, such as the length L1 when the stent is in the compacted state. In some embodiments, the longitudinal length of the stent is a function of the radius of the stent. For example, the longitudinal length of the stent can decrease as the stent radially expands. This is because, as shown, the struts move (e.g., rotate) during radial expansion of the stent. Such movement can result in a decrease in a component length Lx of the struts 22 that is parallel to the longitudinal axis and/or an increase in a component length Ly of the struts 22 that is perpendicular to the longitudinal axis. In certain implementations, in the expanded state, the stent 50 has a longitudinal length of L2, which is less than L1. For example, the ratio of L1 to L2 can be less than or equal to approximately: 1.05, 1.10, 1.15, 1.20, 1.25, 1.30, 1.40, values between the aforementioned values, or otherwise.

FIGS. 25 and 26A-B illustrate details of the compacted structure of FIG. 24A. FIG. 26A shows a cross-section of the tubular structure along line A-A in FIG. 25. FIG. 26B shows a cross-section along line B-B in FIG. 25, which is the compacted structure 50 shown in FIG. 24A. The numbered arrows within FIGS. 26A-B indicate the series or sequence of cells 21 of ring 51. In some embodiments, the rails 26 and/or the struts 22 may be curved circumferentially in the compact configuration. Such a configuration can allow the rails 26 and/or the struts 22 to be positioned closely to a central lumen (such as a compacted balloon catheter) when the stent 20 is in the compacted state, which can reduce the diameter of the stent 20.

As the stent 20 is expanded (e.g., by the balloon), the struts 22 and/or the rails 26 can bend. This can reduce the curvature of such components (e.g., to approximately the circumference of the deployed stent structure). For example, in some embodiments, in the compacted state, the rails 26 and/or the struts 22 are oriented at an angle with respect to a circumferential axis of the stent 20, and in a partially and/or completely expanded state, the rails 26 and/or the struts 22 are oriented generally parallel with the circumferential axis.

FIGS. 27 and 28A-B illustrate details of the expanded structure of FIG. 24C. FIG. 28A shows a cross section of the tubular structure along line A-A in FIG. 27. FIG. 28B shows a cross-section along line B-B in FIG. 27. In certain embodiments, when the stent 20 is in the fully expanded state, some or each of the rails 26 is offset from at least one, or each, of the longitudinally adjacent rails 26. For example, in the embodiment shown, longitudinally adjacent rails 26 are circumferentially spaced apart (e.g., do not overlap in the circumferential direction). In certain implementations, the stent 20 is in the fully expanded state, the arc length of a given rail 26 on the tubular stent does not share a portion of the circumference of the tubular member with the arc length of a longitudinally adjacent rail 26.

As shown in comparing FIGS. 25 and 27, the struts 22 can rotate relative to the longitudinal axis during expansion of the stent. For example, from the fully compacted state to the fully expanded state, some or all of the struts 22 can rotate relative to the longitudinal axis by at least approximately: 60°, 70°, 80°, 90°, 100°, 110°, 120°, values between the aforementioned values, or otherwise. Rotation of the struts 22 can facilitate radial expansion of the circumferential rings formed by the struts, thereby allowing the stent to radially expand.

In some embodiments, the circumferential rings can include non-rotating base portions connected with the struts 22. In the embodiment shown, the base portions are oriented generally parallel with the circumferential axis of the stent. Each of the base portions can be connected with a plurality (e.g., two) of struts. In certain variants, the struts 22 connected with a given base portion rotate about that base portion.

FIGS. 29A-C show three examples of the many possible alternative configurations of the slide and lock braced deformable stent structures. These examples have a spacing of S&L braced cells of 1:3, as in FIG. 22C. However, other embodiments have other spacings, such as 1:2, 1:4, 1:5, 1:6, or otherwise.

FIG. 29A shows an array 52, which is representative of the alternating arrangement of the array 50 in FIGS. 23-28. FIG. 29B shows an array 53 in which the S&L mechanism 30 of adjacent rings 51 are offset from each other. In some embodiments, the offset of the adjacent rings 51 forms a helical pattern around some or all of the circumference of the tubular member. FIG. 29C shows an array 54 in which the S&L mechanism 30 of adjacent rings are staggered to form an alternating pattern, similar to FIG. 29A. In FIGS. 29A and 29B, each ring of cells 51 includes six cells 21. But other numbers of cells per ring are contemplated (e.g., 7, 8, 9, 10, 11, 12, 13, 14, 15, 16, or more). For example, as shown in FIG. 29C, the stent 20 can include 9 rows of cells. In some embodiments, certain adjacent rings of cells 51 have dissimilar numbers of cells 21. For example, a row can have N cells (e.g., 6) and an adjacent row can have N-1 cells (e.g., 5). As shown, the S&L cells can be circumferentially spaced apart from each other by non-S&L cells.

FIGS. 30A-C, 31, and 32 illustrate another embodiment of a tubular stent structure. In some respects, this stent structure is generally similar to the example of FIGS. 24-27 and can include any or all of the features of that stent. For example, the stent of FIGS. 30A-C can include struts 22 that form circumferential rings. The struts 22 rotate relative to the longitudinal axis as the stent expands.

In the embodiment illustrated, there is a spacing of S&L braced cells of 1:2. In some aspects, this is similar to what is shown in FIG. 22D, e.g., one S&L mechanism for each two sequential cells of the ring. FIGS. 30A-B illustrate the compacted to expanded configurations respectively of the embodiment, and are generally similar to FIGS. 24-26, except for the difference in arrangement of the S&L mechanisms 30. As shown, when the stent is in the fully expanded state, some or each of the rails 26 is circumferentially overlapped with a longitudinally adjacent rail 26. For example, in the embodiment shown, longitudinally adjacent rails 26 are circumferentially nested (e.g., overlap in the circumferential direction). In certain implementations, the stent is in the fully expanded state, the arc length of a given rail 26 on the tubular stent shares a portion of the circumference of the tubular member with the arc length of a longitudinally adjacent rail 26.

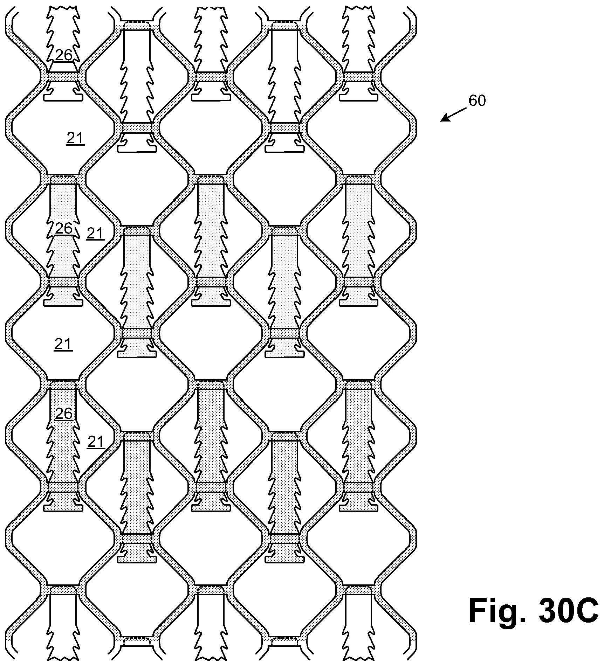

FIG. 32 illustrates another illustrative embodiment of a stent 61, which can include any of the features of any of the other stents described herein. As shown, the stent 61 includes S&L braced cells. The stent 61 can have 1:2 spacing, though many other spacings are contemplated as well. In some embodiments, adjacent S&L mechanisms are arranged in a staggered or alternating pattern (e.g., as in FIGS. 30-31). As illustrated, the S&L cells can be circumferentially adjacent to other S&L cells.

FIGS. 33 and 34 show another embodiment of a stent 62, which can include any of the features of any of the other stents described herein. As shown, the stent 62 includes S&L braced cells. The stent 62 is illustrated with 1:2 spacing, though many other spacings are contemplated as well. In certain variants, adjacent S&L mechanisms are arranged in an undulating pattern, such as zig-zag, chevron, generally sinusoidal, or otherwise. In this example, the chevron pattern changes direction at a "corner" after three adjacent rings 51.

FIGS. 35A-C illustrate three further alternative arrangements (among many) of the S&L braced cells in a stent having 1:2 spacing. As shown, the various embodiments can have different patterns of adjacent S&L mechanisms and/or different numbers of cells in each tubular ring. These examples also show that the tubular stent form may comprise rings 51 that are subdivided into a selected number of cells 21. In some embodiments, such as is shown in FIG. 35A, a stent 63 includes rings 51 of eight cells 21, a S&L mechanism spacing of 1:2, and/or an overall helical pattern of adjacent mechanisms 30. In certain implementations, such as is illustrated in FIG. 35B, a stent 64 has rings 51 of four cells 21, a S&L mechanism spacing of 1:2, and/or an overall helical pattern of adjacent mechanisms 30. In some variants, such as is shown in FIG. 35C, the stent 65 includes rings 51 of four cells 21, a S&L mechanism spacing of 1:2, and/or an overall helical pattern of adjacent mechanisms 30.

FIG. 36 illustrates another embodiment of a stent 70, which can include any of the features of any of the other stents described herein. The stent 70 can include rings of cells having different S&L spacing at different points along its longitudinal extent. For example, the illustrated embodiment has 1:2 spacing at proximal and distal end rings, and 1:3 spacing between end-rings. An end-ring 72 can include, in this example, a ring of six cells 21 with S&L mechanisms 30 at a 1:2 spacing (e.g., three mechanisms 30 in six cells 21). In some embodiments, the interior-rings 73 can each include a ring 73 of six cells 21 with S&L mechanisms 30 at a 1:3 spacing (e.g., two mechanisms 30 in six cells 21). This longitudinally non-uniform spacing can allow the mechanical characteristics to be tailored longitudinally. For example, it may be desired to have a greater crush-strength at the center portions than at the end portions, or vice versa. In some embodiments, the end rings 72 have a greater spacing ratio than some or all of the interior rings 73. This can provide added support for the ends of the stent 70.

FIGS. 37A-B illustrate close-up views of a portion of a further embodiment of a stent 80, which can include any of the features of any of the other stents described herein. FIG. 37A shows the stent 80 in a compacted state and FIG. 37B shows the stent 80 in the expanded state. In some embodiments, the stent 80 includes a ring 81 comprising deformable cells. The ring 81 can comprise one or more (e.g., two) undulating annular backbones (e.g., comprising struts 22) that are connected with a plurality of cross members. As described above, the rails can be connected with certain of the cross members and can slidingly pass through other cross members to provide S&L functionality.

As shown, the ring 81 can be separate from adjacent rings, such as with spaced-apart interconnections between adjacent rings. For example, as illustrated, each ring 81 (which can be the same or similar to the rings of any the forgoing embodiments), can be connected with a longitudinally adjacent ring 81 by a plurality of linking elements 82. The linking elements 82 can be configured to provide longitudinal flexibility and/or can allow the rings 81 to move (e.g., twist or change shape) relative to the adjacent rings. In certain implementations, this allows the stent to elongate longitudinally, or at least decrease in longitudinal length a lesser amount, as the stent expands radially. In some variants, because of the longitudinal flexibility provide by the linking elements 82, the longitudinal length of the stent is not a function of the radius of the stent (unlike in certain other embodiments, such as is shown in FIGS. 24B-C). In other embodiments, as described above, the ring 81 is integrated with a longitudinally adjacent ring 81, such as by sharing common struts 22 or other portions of the rings 81, 81.

FIGS. 38A-B illustrate close-up views of a portion of another embodiment of a stent 85, which can include any of the features of any of the other stents described herein. FIG. 38A shows the stent 85 in a compacted state and FIG. 38B shows the stent 85 in the expanded state. The stent 85 can include rings 81 mechanically connected with linking elements 82. As illustrated, the linking elements 82 can be located in annular portions 83, 84. In some embodiments, the mechanical coupling between adjacent rings is governed in part by the characteristics of linking elements 82 and/or the spacing of linking elements 82. For example, the amount that adjacent rings can move relative to each other can increase as the radial thickness or circumferential width of the linking elements 82 decreases, and/or as the longer the longitudinal length of the linking elements 82 increases. In some embodiments, the linking elements 82 can include serpentine portions, biasing portions, or other portions configured to facilitate at least some independent movement of adjacent ring 81.

Various arrangements of the links 82 between adjacent cell rings are contemplated. In some embodiments, the stent 85 includes adjacent cell rings 81, 81 that are separated by a continuous sequence of linking elements 82, such as is shown in the linking row 83. For example, as shown, the adjacent cell rings 81, 81 can have a peak-to-peak configuration (e.g., the peaks on one of the rings are generally circumferentially aligned with the peaks on the adjacent ring, and/or the valleys on one of the rings are generally circumferentially aligned with the valleys on the adjacent ring). As illustrated, the linking elements 82 can extend between each set of peaks and valleys.

In certain embodiments, the cell ring 81 includes first and second undulating annular supports. In certain embodiments, the undulating annular supports comprise the struts 22. As shown, in certain variants, the first and second undulating annular supports can be about 180° out of phase circumferentially. This can result in a peak-to-peak configuration, which can facilitate flexibility and/or deformability of the stent. For example, the peaks of the first and second undulating annular supports can be generally circumferentially aligned and/or the valleys of the first and second undulating annular supports can be generally circumferentially aligned. In some embodiments, the first undulating annular support is about in-phase circumferentially with the second undulating annular support. This can result in the peaks of one of the first and second undulating annular supports being generally circumferentially aligned with the valleys of the other of the first and second undulating annular supports. This can facilitate nesting of the cell rings when the stent is in the compacted state.

In some embodiments, the stent 85 includes adjacent cell rings 81, 81 that are separated by discontinuous (e.g., spaced-apart) sequence of the linking elements 82, such as is shown in the linking row 84. For example, as illustrated, the linking elements 82 can extend between every other set of peaks and valleys. In some embodiments, the every third set of linking elements 82 can extend between every third, fourth, fifth, or other set of peaks and valleys. As illustrated, in certain variants with a discontinuous sequence of the linking elements 82, adjacent linking rows 84 can be circumferentially offset. This can result in the linking elements 82 being staggered along the longitudinal axis. In some variants, the linking rows 84 are generally circumferentially aligned. This can form a longitudinal row of the linking elements 82. In various embodiments, the linking rows 84 may have additional discontinuities and/or differing circumferential offsets.

In some implementations, the stent 81 includes adjacent cell rings 81, 81 with both continuous sequence of the linking elements 82 and discontinuous sequence of the linking elements 82. For example, as shown in FIGS. 38A and 38B, certain of the adjacent cell rings 81, 81 are separated by a continuous sequence of the links 82 and certain of the adjacent cell rings 81, 81 separated by discontinuous linking elements 82.

FIG. 39 shows an example of another embodiment of a stent 55, which can include any of the features of any of the other stents described herein. In some embodiments, the stent 55 has proximal and distal ends that are bounded by a sequence or row 86 of S&L mechanisms 30'. As shown, in certain variants, the S&L mechanisms 30' protrude longitudinally beyond a terminal cell ring 51 (e.g., the cell ring at a longitudinal end of the stent 55). The end cell ring 51 can include support members 24' and 25', which can extend generally longitudinally and/or can be cantilevered. A rail 26 can be connected to the support members 24' and can be slidably engaged with the other support member 25'. This can provide slide and lock functionality for the end ring 51, and thus the stent 55. In some variants, the rail member 26 engages one or more of the struts 22' and/or an apex or valley between the struts 22'.

FIGS. 40-42 exemplify certain further stent features. These features can be incorporated into any of the embodiments described in FIGS. 15-39, or in any of the other embodiments described herein, as desired or to achieve particular device characteristics. FIG. 40 illustrates an embodiment wherein one or more cells of a ring of cells is further subdivided by having multiple corners or undulation of the struts 91 bordering the cell 21'. In this example, the modified cell is included in the S&L mechanism, or it may be included in other cells 21' of the ring of cells. Also shown in FIG. 40 is a non-S&L cross-member 92, which can be included in selected cells if desired to modify mechanical characteristics. For example, the non-S&L cross-member 92 can provide additional structural support, can increase the crush strength of the stent, and/or can limit relative longitudinal movement of the undulating rings that form the longitudinal sides of the cells.

FIG. 41 illustrates another illustrative embodiment. As shown, this embodiment can be similar in some respects to the embodiment shown in FIGS. 37-38. However, rather than having linking elements 82, intervening pairs of rings 96 of struts 97 (between cell rings 81 having S&L mechanisms) are integrated or connected, so as to have common joint portions 95 binding the adjacent strut rings. In some embodiments, longitudinally adjacent undulating rings are integrated or connected (e.g., share a common structural portion). The openings in the intervening rows are designated 21".

FIG. 42 illustrates another illustrative embodiment. In some implementations, rings of struts are arranged in a peak-to-valley pattern (see FIG. 20B). For example, the peaks and valleys of one of the rings of struts can be substantially in phase with the peaks and valleys of a longitudinally adjacent ring of struts. As shown, this can result in cells 21"', which have an undulating configuration, rather than diamond configuration as in certain other embodiments.

As also shown in FIG. 42, in some variants, the direction of the toothed rail members reverses in adjacent rings of cells. For example, as shown, a rail 26 can extend in a generally circumferential direction and a rail 26' in a longitudinally adjacent ring of cells can extend in a generally opposite circumferential direction. In some embodiments, the rings of cells include struts 99 and cross members 24" and 25", which can be configured to support the S&L elements (e.g., connection area 34, slot 35, and rail 26 or 26').

### Materials for deformable stents having slide and lock elements

The stent embodiments illustrated in FIGS. 21 through 41 may be constructed of known polymer, metal, alloy, composite or reinforced compositions as used in the medical device art, both in biodegradable and persistent types. See, for example, the stent material compositions described in the following patents and patent application publications: US Patent and Publication Nos. 7,727,272; 6,287,332; 7,572,287; 2005-0232971; and 2007-0050018.

Furthermore, different components of the stent embodiments described herein may be formed of different materials. For example, the deformable strut rings may be of metallic composition (e.g., a biodegradable metal), while the toothed rail may be formed of a polymer composition or polymer composite. Alternatively, the deformable strut rings may be formed of a polymer composition or polymer composite, while the toothed rail may be metallic in composition.

The figures above are not intended to be limiting as to particular dimensions or relative sizes of elements. With respect to distances and dimensions of the components of stent 100, it should be noted relatively smaller thicknesses and dimensions of either or both of portions of backbones 101 and/or 102, or of rails 103, may result in higher forces and moments, such as bending moments, in a given medical application, such as a coronary artery stent deployment. Such forces and moments depend on a number of different design aspects as well as the particular stent deployment operation itself (e.g., balloon pressure, nature and size of lesion, etc). However, medical polymer structures providing both biodegradability and radiopacity, combined with high strength and fatigue resistance, are enabled by the polymeric materials, monomers and processes disclosed in US Patent No. 8,252,887, and in pending US Patent Application No. 13/757,752 filed February 2, 2013 (not yet published), both assigned to Rutgers State University. Such materials of high strength and fatigue resistance may support stent designs having substantially smaller dimensions.

### Lamination Manufacturing Process Embodiments

Stents in accordance with embodiments can be fabricated or created using a wide variety of manufacturing methods, techniques and procedures. These include, but are not limited to, laser processing, milling, stamping, forming, casting, molding, bonding, welding, adhesively fixing, and the like, among others.

In some embodiments, stent features and mechanisms can be created in a generally two dimensional geometry and further processed, for example by utilizing, but not limited to, bonding, lamination and the like, into three dimensional designs and features. In other embodiments, stent features and mechanisms can be directly created into three dimensional shapes, for example by utilizing, but not limited to, processes such as injection molding and the like.

In certain embodiments, stents can be fabricated by using an injection molding process, technique or method. For example, an injection molding process or the like, among others, can be used to form stent rows as integral units. The axially extending rows can then be connected and rolled into a tubular form in the compacted state.

In some embodiments, a lamination stack can used to fabricate the stent rows by a lamination process in accordance with one embodiment. The axially extending rows can then be connected and rolled into a tubular form in the compacted state.

The lamination stack, in some embodiments, generally can comprise three sheets or pallets which can have the desired features formed thereon, for example, by laser cutting, etching and the like. The pallets can be aligned and joined, for example, by bonding, welding and the like to form a unit. The excess material (e.g., side and end rails) can be removed to form the stent rows. The pallets can include various circumferentially nesting features such as male and female articulating and/or ratcheting designs to control and limit the diameter in compacted and fully deployed states.

### Metal Stents and Methods of Manufacturing

Preferred materials for making the stents in accordance with some embodiments include cobalt chrome, 316 stainless steel, tantalum, titanium, tungsten, gold, platinum, iridium, rhodium and alloys thereof or pyrolytic carbon. In still other alternative embodiments, the stents can be formed of a corrodible material, for instance, a magnesium alloy. Although preferred stent embodiments have been described as being conventional balloon expandable stents, those skilled in the art will appreciate that stent constructions according to embodiments can also be formed from a variety of other materials to make a stent crush-recoverable. For example, in alternative embodiments, such as self expandable stents, shape memory alloys that allow for such, such as Nitinol and Elastinite®, can be used in accordance with embodiments.

Preferred methods of forming the individual elements from metal sheets can be laser cutting, laser ablation, die-cutting, chemical etching, plasma etching and stamping and water jet cutting of either tube or flat sheet material or other methods known in the art which are capable of producing high-resolution components. The method of manufacture, in some embodiments, depends on the material used to form the stent. Chemical etching provides high-resolution components at relatively low price, particularly in comparison to high cost of competitive product laser cutting. Some methods allow for different front and back etch artwork, which could result in chamfered edges, which can be desirable to help improve engagements of lockouts. Further one can use plasma etching or other methods known in the art which are capable of producing high-resolution and polished components. The embodiments disclosed herein are not limited to the means by which stent or stent elements can be fabricated.

Once the base geometry is achieved, the elements can be assembled numerous ways. Tack-welding, adhesives, mechanical attachment (snap-together and/or weave together), and other art-recognized methods of attachment, can be used to fasten the individual elements. Some methods allow for different front and back etch artwork, which could result in chamfered edges, which can be desirable to help improve engagements of lockouts. In one preferred method of manufacture, the components of the stent can be heat set at various desired curvatures. For example, the stent can be set to have a diameter equal to that of the deflated balloon, as deployed, at a maximum diameter, or greater than the maximum diameter. In yet another example, elements can be electropolished and then assembled, or electropolished, coated, and then assembled, or assembled and then electropolished.

### Polymeric Stents

While metal stents possess certain desirable characteristics, the useful lifespan of a stent is estimated to be in the range of about 6 to 9 months, the time at which in-stent restenosis stabilizes and healing plateaus. In contrast to a metal stent, a bioresorbable stent cannot outlive its usefulness within the vessel. Moreover, a bioresorbable stent could potentially be used to deliver a greater dose of a therapeutic agent, deliver multiple therapeutic agents at the same time or at various times of its life cycle, to treat specific aspects or events of vascular disease. Additionally, a bioresorbable stent can also allow for repeat treatment of the same approximate region of the blood vessel. Accordingly, there remains an important unmet need to develop temporary (i.e., bioresorbable and/or radiopaque) stents, wherein the polymeric materials used to fabricate these stents can have the desirable qualities of metal (e.g., sufficient radial strength and radiopacity, etc.), while circumventing or alleviating the many disadvantages or limitations associated with the use of permanent metal stents.

In one preferred embodiment, the stent can be formed from biocompatible polymers that are bio-resorbable (e.g., bio-erodible or bio-degradable). Bio-resorbable materials can be preferably selected from the group consisting of any hydrolytically degradable and/or enzymatically degradable biomaterial. Examples of suitable degradable polymers include, but are not limited to, polyhydroxybutyrate /polyhydroxyvalerate copolymers (PHV/PHB), polyesteramides, polylactic acid, polyglycolic acid, lactone based polymers, polycaprolactone, poly(propylene fumarate-co-ethylene glycol) copolymer (aka fumarate anhydrides), polyamides, polyanhydride esters, polyanhydrides, polylactic acid/polyglycolic acid with a calcium phosphate glass, polyorthesters, silk-elastin polymers, polyphosphazenes, copolymers of polylactic acid and polyglycolic acid and polycaprolactone, aliphatic polyurethanes, polyhydroxy acids, polyether esters, polyesters, polydepsidpetides, polysaccharides, polyhydroxyalkanoates, and copolymers thereof. For additional information, see US Patent Nos. 4,980,449, 5,140,094, and 5,264,537.

In one mode, the degradable materials can be selected from the group consisting of poly(glycolide-trimethylene carbonate), poly(alkylene oxalates), polyaspartimic acid, polyglutarunic acid polymer, poly-p-dioxanone, poly-.beta.-dioxanone, asymmetrically 3,6-substituted poly-1,4-dioxane-2,5-diones, polyalkyl-2-cyanoacrylates, polydepsipeptides (glycine-DL-lactide copolymer), polydihydropyranes, polyalkyl-2-cyanoacrylates, poly-.beta.-maleic acid (PMLA), polyalkanotes and poly-.beta.-alkanoic acids. There are many other degradable materials known in the art. (*See e.g*., Biomaterials Science: An Introduction to Materials in Medicine (29 July, 2004) Ratner, Hoffman, Schoen, and Lemons; and Atala, A., Mooney, D. Synthetic Biodegradable Polymer Scaffolds. 1997 Birkhauser, Boston).

Further still, in a more preferred embodiment, the stents can be formed of a polycarbonate material, such as, for example, tyrosine-derived polycarbonates, tyrosine-derived polyarylates, tyrosine-derived diphenol monomers, iodinated and/or brominated tyrosine-derived polycarbonates, iodinated and/or brominated tyrosine-derived polyarylates. For additional information, see US Patent Nos. 5,099,060, 5,198,507, 5,587,507, which was re-issued in RE37,160, 5,670,602, which was re-issued in RE37,795, 5,658,995, 6,048,521, 6,120,491, 6,319,492, 6,475,477, 5,317,077, and 5,216,115, and US Publication No. 2008-0152,690. In another preferred embodiment, the polymer can be any of the biocompatible, bioabsorbable, radiopaque polymers disclosed in: US Patent Nos. 8,034,365; 8,008,528; 7,939,611; 7,473,417; 7,250,154; 7,056,493; and 6,852,308; and in US Publication Nos. 2010-0131037; 2006-0204440; 2006-0182779; 2005-0106119; and 2006-0115449.

In some embodiments, the polymer can be any of the biocompatible, bioabsorbable, radiopaque polymers disclosed in US Patent and Publication Nos. 8,252,887; 2010-0228343; 2012-0197001; 2012-0178885; 2012-0189713; 2012-0226013; 2012-0108677; and 2013-203713.

Natural polymers (biopolymers) include any protein or peptide. Preferred biopolymers can be selected from the group consisting of alginate, cellulose and ester, chitosan, collagen, dextran, elastin, fibrin, gelatin, hyaluronic acid, hydroxyapatite, spider silk, cotton, other polypeptides and proteins, and any combinations thereof.

In yet another alternative embodiment, shape-shifting polymers can be used to fabricate stents constructed according to embodiments. Suitable shape-shifting polymers can be selected from the group consisting of polyhydroxy acids, polyorthoesters, polyether esters, polyesters, polyamides, polyesteramides, polydepsidpetides, aliphatic polyurethanes, polysaccharides, polyhydroxyalkanoates, and copolymers thereof. For addition disclosure on bio-degradable shape-shifting polymers, see US Patent Nos. 6,160,084 and 6,284,862. For additional disclosure on shape memory polymers, see US Patent Nos. 6,388,043 and 6,720,402. Further the transition temperature can be set such that the stent can be in a compacted condition at a normal body temperature. However, with the application of heat during stent placement and delivery, such as via a hot balloon catheter or a hot liquid (e.g., saline) perfusion system, the stent can expand to assume its final diameter in the body lumen. When a thermal memory material is used, it can provide a crush-recoverable structure.

Further still, stents can be formed from biocompatible polymers that are biostable (e.g., non-degrading and non-erodible). Examples of suitable non-degrading materials include, but are not limited to, polyurethane, Delrin, high density polyethylene, polypropylene, and poly(dimethyl siloxane).

In some embodiments, the layers can comprise or contain any example of thermoplastics, such as the following, among others: fluorinated ethylene-propylene, poly(2-hydroxyethyl methacrylate) (aka pHEMA), poly(ethylene terephthalate) fiber (aka Dacron®) or film (Mylar®), poly(methyl methacrylate) (aka PMMA), Poly(tetraflouroethylene) (aka PTFE and ePTFE and Gore-Tex®), poly(vinyl chloride), polyacrylates and polyacrylonitrile (PAN), polyamides (aka Nylon), polycarbonates and polycarbonate urethanes, polyethylene and poly(ethylene-co-vinyl acetate), polypropylene, polystyrene, polysulphone, polyurethane and polyetherurethane elastomers such as Pellethane® and Estane®, Silicone rubbers, Siloxane, polydimethylsiloxane (aka PDMS), Silastic®, Siliconized Polyurethane.

The polymer(s) utilized in embodiments of the stent can be fabricated according to any variety of processes, such as those discussed in US Patent Application Nos. 60/852,471 and 60/852,513, and US Patent Nos. 5,194,570, 5,242,997, 6,359,102, 6,620,356, and 6,916,868.

### Methods of Manufacturing and Assembling Polymeric Stents

Where plastic and/or degradable materials are used, the elements can be made using laser ablation with a screen, stencil or mask; solvent casting; forming by stamping, embossing, compression molding, centripetal spin casting and molding; extrusion and cutting, three-dimensional rapid prototyping using solid free-form fabrication technology, stereolithography, selective laser sintering, or the like; etching techniques comprising plasma etching; textile manufacturing methods comprising felting, knitting, or weaving; molding techniques comprising fused deposition modeling, injection molding, room temperature vulcanized molding, or silicone rubber molding; casting techniques comprising casting with solvents, direct shell production casting, investment casting, pressure die casting, resin injection, resin processing electroforming, or injection molding or reaction injection molding. Certain preferred embodiments with the disclosed polymers can be shaped into stents via combinations of two or more thereof, and the like.

Such processes can further include two-dimensional methods of fabrication such as cutting extruded sheets of polymer, via laser cutting, etching, mechanical cutting, or other methods, and assembling the resulting cut portions into stents, or similar methods of three-dimensional fabrication of devices from solid forms. For additional information, see US Patent No. 6,749,584.

Stents of the preferred embodiment can be manufactured with elements prepared in full stent lengths or in partial lengths of which two or more are then connected or attached. If using partial lengths, two or more can be connected or attached to comprise a full length stent. In this arrangement the parts can be assembled to give rise to a central opening. The assembled full or partial length parts and/or modules can be assembled by inter-weaving them in various states, from a compacted state, to a partially expanded state, to an expanded state.

Further, elements can be connected or attached by solvent or thermal bonding, or by mechanical attachment. If bonding, preferred methods of bonding comprise the use of ultrasonic radiofrequency or other thermal methods, and by solvents or adhesives or ultraviolet curing processes or photoreactive processes. The elements can be rolled by thermal forming, cold forming, solvent weakening forming and evaporation, or by preforming parts before linking.

Rolling of the flat series of module(s) to form a tubular member can be accomplished by any means known in the art, including rolling between two plates, which can be each padded on the side in contact with the stent elements. One plate can be held immobile and the other can move laterally with respect to the other. Thus, the stent elements sandwiched between the plates can be rolled about a mandrel by the movement of the plates relative to one another. Alternatively, 3-way spindle methods known in the art can also be used to roll the tubular member. Other rolling methods that can be used in accordance with certain embodiments include those used for "jelly-roll" designs, as disclosed for example, in US Patent Nos. 5,421,955, 5,441,515, 5,618,299, 5,443,500, 5,649,977, 5,643,314 and 5,735,872.

The construction of the slide-and-lock stents in these fashions can provide a great deal of benefit over the prior art. The construction of the locking mechanism can be largely material-independent. This allows the structure of the stent to comprise high strength materials, not possible with designs that require deformation of the material to complete the locking mechanism. The incorporation of these materials will allow the thickness required of the material to decrease, while retaining the strength characteristics of thicker stents. In preferred embodiments, the frequency of catches, stops or teeth present on selected circumferential elements can prevent unnecessary recoil of the stent subsequent to expansion.

### Radiopacity

Traditional methods for adding radiopacity to a medical product include the use of metal bands, inserts and/or markers, electrochemical deposition (i.e., electroplating), or coatings. The addition of radiopacifiers (i.e., radiopaque materials) to facilitate tracking and positioning of the stent could be accommodated by adding such an element in any fabrication method, by absorbing into or spraying onto the surface of part or all of the device. The degree of radiopacity contrast can be altered by element content.

For plastics and coatings, radiopacity can be imparted by use of monomers or polymers comprising iodine or other radiopaque elements, i.e., inherently radiopaque materials. Common radiopaque materials include barium sulfate, bismuth subcarbonate, and zirconium dioxide. Other radiopaque elements include: cadmium, tungsten, gold, tantalum, bismuth, platinum, iridium, and rhodium. In one preferred embodiment, a halogen such as iodine and/or bromine can be employed for its radiopacity and antimicrobial properties.

### Multi-Material Vascular Prosthesis

In still other alternative embodiments, various materials (e.g., metals, polymers, ceramics, and therapeutic agents) can be used to fabricate stent embodiments. The embodiments can comprise: 1) differentially layered materials (through stacking in the vertical or radial axis) to create a stack of materials (materials can be stacked in any configuration, e.g., parallel, staggered, etc.); 2) spatially localized materials which can vary along the long axis and/or thickness of the stent body; 3) materials that are mixed or fused to create a composite stent body (e.g., whereby a therapeutic agent(s) is within the stent body with a polymer); 4) embodiments whereby a material can be laminated (or coated) on the surface of the stent body (*see* Stent Surface Coatings with Functional Properties as well as see Therapeutic Agents Delivered by Stents); and, 5) stents comprised of 2 or more parts where at least one part can be materially distinct from a second part, or any combination thereof.

The fashioning of a slide-and-lock multi-material stent can have between two or more materials. Thickness of each material can vary relative to other materials. This approach as needed or desired allows an overall structural member to be built with each material having one or more functions contributing towards enabling prosthesis function which can include, but is not limited to: 1) enabling mechanical properties for stent performance as defined by ultimate tensile strength, yield strength, Young's modulus, elongation at yield, elongation at break, and Poisson's ratio; 2) enabling the thickness of the substrate, geometrical shape (e.g., bifurcated, variable surface coverage); 3) enabling chemical properties of the material that bear relevance to the materials performance and physical state such as rate of degradation and resorption (which can impact therapeutic delivery), glass transition temperature, melting temperature, molecular weight; 4) enabling radiopacity or other forms of visibility and detection; 5) enabling radiation emission; 6) enabling delivery of a therapeutic agent (*see* Therapeutic Agents Delivered by Stents); and 7) enabling stent retention and/or other functional properties (*see* Stent Surface Coatings with Functional Properties).

In some embodiments, the materials can comprise load-bearing properties, elastomeric properties, mechanical strength that can be specific to a direction or orientation e.g., parallel to another material and/or to the long axis of the stent, or perpendicular or uniform strength to another material and/or stent. The materials can comprise stiffeners, such as the following, boron or carbon fibers, pyrolytic carbon. Further, stents can be comprised of at least one re-enforcement such a fibers, nanoparticles or the like.

In another preferred mode of some embodiments, the stent can be made, at least in part, from a polymeric material, which can be degradable. The motivation for using a degradable stent can be that the mechanical support of a stent can only be necessary for several weeks. In some embodiments, bioresorbable materials with varying rates of resorption can be employed. For additional information, see US Published Patent and Application Nos. 7,473,417 and 2006-0034,769. Degradable polymeric stent materials can be particularly useful if it also controls restenosis and thrombosis by delivering pharmacologic agents. Degradable materials can be well suited for therapeutic delivery *(see* Therapeutic Agents Delivered by Stents).

In some embodiments, the materials can comprise or contain any class of degradable polymer as previously defined. Along with variation in the time of degradation and/or resorption the degradable polymer can have other qualities that are desirable. For example, in some embodiments the materials can comprise or contain any example of natural polymers (biopolymers) and/or those that degrade by hydrolytic and/or enzymatic action. In some embodiments, the material can comprise or contain any example of hydrogels that can or cannot be thermally reversible hydrogels, or any example of a light or energy curable material, or magnetically stimulateable (responding) material. Each of these responses can provide for a specific functionality.

In some embodiments, the materials can comprise or be made from or with constituents which can have some radiopaque material alternatively, a clinically visible material which can be visible by x-ray, fluoroscopy, ultrasound, MRI, or Imatron Electron Beam Tomography (EBT).

In some embodiments, one or more of the materials can emit predetermined or prescribed levels of therapeutic radiation. In one embodiment, the material can be charged with beta radiation. In another embodiment, the material can be charged with Gamma radiation. In yet another embodiment, the material can be charged with a combination of both Beta and Gamma radiation. Stent radioisotopes that can be used include, but are not limited to, 103Pd and 32P (phosphorus-32) and two neutron-activated examples, 65Cu and 87Rb2O, (90)Sr, tungsten-188 (188).

In some embodiments, one or more of the materials can comprise or contain a therapeutic agent. The therapeutic agents can have unique, delivery kinetics, mode of action, dose, half-life, purpose, et cetera. In some embodiments, one or more of the materials comprise an agent which provides a mode and site of action for therapy for example by a mode of action in the extracellular space, cell membrane, cytoplasm, nucleus and/or other intracellular organelle. Additionally an agent that serves as a chemoattractant for specific cell types to influence tissue formation and cellular responses for example host-biomaterial interactions, including anti-cancer effects. In some embodiments, one or more of the materials deliver cells in any form or state of development or origin. These could for example be encapsulated in a degradable microsphere, or mixed directly with polymer, or hydrogel and serve as vehicle for pharmaceutical delivery. Living cells could be used to continuously deliver pharmaceutical type molecules, for instance, cytokines and growth factors. Nonliving cells can serve as a limited release system. For additional concepts of therapeutic delivery, see the section entitled: Therapeutic Agents Delivered by Stents.

### Therapeutic Agents Delivered by Stents

In another preferred variation, the stent further can comprise an amount of a therapeutic agent (as previously defined for a pharmaceutical agent and/or a biologic agent) sufficient to exert a selected therapeutic effect. The material of at least a portion of the stent itself can comprise at least one therapeutic agent, or at least one therapeutic agent can be added to the stent in a subsequent forming process or step. In some preferred embodiments of the stent (e.g., polymer stents and multi-material stents), the therapeutic agent can be contained within the stent as the agent is blended with the polymer or admixed by other means known to those skilled in the art.

For example, one or more therapeutic agents can be delivered through a multi-material vascular prosthesis. In some embodiments, the entire stent can be formed from materials comprising one or more therapeutic agents. In some embodiments, portions of the stent, such as individual components thereof, can comprise materials comprising one or more therapeutic agents. In such embodiments, it is contemplated that the therapeutic agent(s) can be released as the stent material degrades.

For example, the therapeutic agent can be embedded or impregnated into the film by means of a combination of solvent casting and thermal pressing. In such a method, the film can be formed from a mixture of the polymer and the therapeutic agent (20% solids polymer, for example poly(90%DTE-co-10%DT carbonate), which can be made with 1% rapamycin in dichloromethane). Once this mixture is prepared, the film can be cast using a doctor blade. Alternatively, the film can be formed by using a mechanical reverse roll coater or other solvent-based film caster. Once the film is cast, the solvent can be evaporated off using a vacuum oven, e.g., for a period of time and at a temperature suitable for the polymer and drug, such as at 40°C for at least 20 hours. Once the film is dried, it can be thermally pressed, e.g., at a temperature of 100°C between two heated platens of a hydraulic press. This allows the potency of the drug to be retained.

In addition, the therapeutic agent can be embedded or impregnated into the film using only a solvent or by spin casting. Once a therapeutic agent is selected, one needs to determine if the solvent is compatible with the agent and the polymer chosen. The objective is to prepare a suitable sprayable suspension. Additionally, the stability of the drug can be measured such that the therapeutic agent can remain active while in the coating as well under physiological conditions once released from the film. This can be determined by those skilled in the art who conduct standard in vitro elution studies *(see* Dhanikula et al., Development and Characterization of Biodegradable Chitosan Films for Local Delivery of Paclitaxel, The AAPS Journal, 6 (3) Article 27 (2004); and Kothwala et al., Paclitaxel Drug Delivery from Cardiovascular Stent, Trends in Biomaterials & Artificial Organs, Vol. 19(2), 88-92 (2006)) of agent embedded films and through the use of analytical methods such as HPLC methods (*see* Dhanikula et al., Development and Characterization of Biodegradable Chitosan Films for Local Delivery of Paclitaxel; and Kothwala et al., Paclitaxel Drug Delivery from Cardiovascular Stent) to detect the purity of the drug.

In some embodiments, at least one therapeutic agent can be added to the stent and/or its components after the formation of the stent and/or its components. For example, at least one therapeutic agent can be added to individual stent components, through a coating process or otherwise. The addition of at least one therapeutic agent can occur before or after cutting or lasing of the stent components. In another example, at least one therapeutic agent can also be added to at least a portion of the stent after partial or full assembly thereof, through a coating process or otherwise. In some embodiments of the stent, the therapeutic agent can be delivered from a polymer coating on the stent surface. In other preferred embodiments of the stent, a therapeutic agent can be localized in or around a specific structural aspect of the device.

For example, the therapeutic agent can be delivered from a polymer coating on the stent surface. Thus, the stent can be made by applying the therapeutic agent to a stent component before the stent is assembled or formed. In this regard, the stent component can be created from a polymer sheet, such as a flat polymer film. Thus, at least one stent component can be separated from a remainder or excess portion of the film either before or after the therapeutic agent has been applied to the stent component and/or film. After the therapeutic agent is applied and the stent component is separated from the film, the stent component can be assembled (and in some embodiments, with other stent components) to form a stent therefrom.

In some embodiments, the stent can be prepared with the following preparation method. The stent can be initially prepared by creating a pattern of a stent component on a flat polymer film. The creation of the pattern on the film can occur before or after application of a therapeutic agent thereto, as discussed below. The pattern of the stent component can be created on the film such that the stent component can be detached from the film when desired. In some embodiments, the pattern can be created using a laser to lase the pattern onto the film. Additionally, the lased pattern can be of any given stent component design, such as that used in a slide and lock stent design. After the pattern is created on the film, the entire film can be cleaned. For example, if the therapeutic agent has not yet been applied to the film, the entire lased film can be immersed into a cleaning solution that is compatible with the specific type of polymer from which the film is made. The cleaned film can then be dried, for example, by being blown and oven dried.

A coating formulation can be prepared by dissolving or dispersing the polymer and the therapeutic agent(s) of choice and solvent(s) or other compatible excipient(s) using a calculated amount of each component to achieve the desired concentration. The coating formulation can then be applied to the lased polymer film using one or more coating methods. For example, the film may be coated by means of spraying, dipping, or other coating methods. Additionally cross-linking reagents may also be used to prepare a coating.

In a spraying coating method, the lased polymer films can be coated with the coating formulation by first mounting the cleaned dried films into a spray apparatus. The coating formulation can then be sprayed onto the film, and the film can be rotated 180 degrees such that the other side can be coated if desired. This method can allow for coating of one or both sides of the stent component(s). This method can also allow one to apply different therapeutic agents per side of the lased film and/or stent component and to selectively coat regions thereof. The method can further allow one to coat multiple drugs per film and/or stent component. Alternative coating methods can allow for other similar benefits.

For example, a therapeutic agent can be coated onto a film or stent component as in the following illustration. First, the therapeutic agent in this example is a Polymer-Paclitaxel Formulation, such as a 0.5% [25%Paclitaxel/75% Poly(86.75%I2DTE-co-10%I2DT-co-3.25%PEG2000 carbonate)] in tetrahydrofuran (THF), which can be prepared using an analytical balance. In order to do so, one must first weigh 0.0150g of Paclitaxel into a tared vial. Then weigh 0.0450g of polymer into another vial. Next, weigh 11.940g of THF into each vial. Shake the vials on a laboratory shaker, such as a Roto-genie, for at least one hour. In this example, coating can be achieved using a spray gun apparatus, such as an air brush (*see* Westedt, U., Biodegradable Paclitaxel-loaded Nanoparticles and Stent Coatings as Local Delivery Systems for the Prevention of Restenosis - Dissertation, Marburg/Lahn (2004), and Berger, H.L. Using Ultrasonic Spray Nozzles to Coat Drug-Eluting Stents, Medical Device Technology (2006)). Typically, the spray gun apparatus should first be cleaned with THF. In order to do so, a syringe can be filled with at least 10 ml of THF. The syringe can then be attached to a spray line attached to the spray gun. Gradually, the 10 ml of THF can be pushed from the syringe into the spray gun without N2 pressure. This can be repeated as necessary to ensure that the line is washed clean. The syringe pump can then be set up with the syringe containing the Polymer-Paclitaxel Formulation.

Next, a film, which can be either lased or unlased, can be placed into a hooded environment and mounted or clipped into a holder. If necessary, the surfaces of the film can be cleaned of lint and dust using a pure air or gas source or equivalent. For consistent coating quality, the film can be programmed to move at a set rate (distance and speed) relative to a spray stream by integrating the film holder apparatus with a motion control system. Manual coating without the motion control can also be used to achieve a coating. The spray gun can also be set to direct the spray to only a given location to control coating distribution.

In some embodiments, to coat both sides of the film uniformly, the spray cycle can start with the spray hitting at the bottom corner of the film, and the motion control should move the film incrementally as it traverses back and forth in front of the spray nozzle. The system can then move the film back to the start position so the spray is directed at the bottom. The film holder can be turned 180 degrees and the cycle can be repeated to coat the second side. After coating, the film holder can be removed with the film and the film can be dried in a vacuum oven at a temperature suitable for the drug and polymer, e.g., 25° ± 5°C for at least 20 hours.

Other methods and teachings related to impregnation or coating processes are found in the following references: Westedt, U., Biodegradable Paclitaxel-loaded Nanoparticles and Stent Coatings as Local Delivery Systems for the Prevention of Restenosis - Dissertation, Marburg/Lahn (2004); Berger, H.L. Using Ultrasonic Spray Nozzles to Coat Drug-Eluting Stents, Medical Device Technology (2006); Dhanikula et al., Development and Characterization of Biodegradable Chitosan Films for Local Delivery of Paclitaxel, The AAPS Journal, 6 (3) Article 27 (2004); and Kothwala et al., Paclitaxel Drug Delivery from Cardiovascular Stent, Trends in Biomaterials & Artificial Organs, Vol. 19(2), 88-92 (2006).

After the film is coated using a given coating method, the film can be given time to dry. Once dried, the lased, coated stent component(s) can be separated from the remainder of the film. Care should be taken to not disturb the surfaces of the coated stent component(s) when being detached from the film and assembled or knitted together to form a three-dimensional cylindrical stent.

In another preferred variation the therapeutic agent can be delivered by means of a non-polymer coating. In other preferred embodiments of the stent, the therapeutic agent can be delivered from at least one region or one surface of the stent. The therapeutic agent can be chemically bonded to the polymer or carrier used for delivery of the therapeutic from at least one portion of the stent and/or the therapeutic can be chemically bonded to the polymer that can comprise at least one portion of the stent body. In some embodiments, a polymer can be used as a component of the coating formulation. Accordingly, the coating can essentially bond directly to a clean lased film and/or stent component, which can also be comprised of a polymer. Such an embodiment of the method can provide for a seamless interface between the coating and the lased film and/or stent component. Further, in another embodiment, more than one therapeutic agent can be delivered.

The amount of the therapeutic agent can be preferably sufficient to inhibit restenosis or thrombosis or to affect some other state of the stented tissue, for instance, heal a vulnerable plaque, and/or prevent rupture or stimulate endothelialization or limit other cell types from proliferating and from producing and depositing extracellular matrix molecules. The agent(s) can be selected from the group consisting of antiproliferative agents, anti-inflammatory, anti-matrix metalloproteinase, and lipid lowering, cholesterol modifying, anti-thrombotic and antiplatelet agents, in accordance with preferred embodiments. For vascular stent applications, some of these preferred anti-proliferative agents that improve vascular patency include without limitation paclitaxel, Rapamycin, ABT-578, Biolimus A9, everolimus, dexamethasone, nitric oxide modulating molecules for endothelial function, tacrolimus, estradiol, mycophenolic acid, C6-ceramide, actinomycin-D and epothilones, and derivatives and analogs of each.

Some of these preferred agents act as an antiplatelet agent, antithrombin agent, compounds to address other pathologic events and/or vascular diseases. Various therapeutic agents can be classified in terms of their sites of action in the host: agents that exert their actions extracellularly or at specific membrane receptor sites, those that act on the plasma membrane, within the cytoplasm, and/or the nucleus.

In addition to the aforementioned, therapeutic agents can include other pharmaceutical and/or biologic agents intended for purposes of treating body lumens other than arteries and/or veins). Therapeutic agents can be specific for treating nonvascular body lumens such as digestive lumens (e.g., gastrointestinal, duodenum and esophagus, biliary ducts), respiratory lumens (e.g., tracheal and bronchial), and urinary lumens (e.g., urethra). Additionally such embodiments can be useful in lumens of other body systems such as the reproductive, endocrine, hematopoietic and/or the integumentary, musculoskeletal/orthopedic and nervous systems (including auditory and ophthalmic applications); and finally, stent embodiments with therapeutic agents can be useful for expanding an obstructed lumen and for inducing an obstruction (e.g., as in the case of aneurysms).

Therapeutic release can occur by controlled release mechanisms, diffusion, interaction with another agent(s) delivered by intravenous injection, aerosolization, or orally. Release can also occur by application of a magnetic field, an electrical field, or use of ultrasound.

### Stent Surface Coatings with Functional Properties

In addition to stents that can deliver a therapeutic agent, for instance delivery of a biological polymer on the stent such as a repellant phosphorylcholine, the stent can be coated with other bioresorbable polymers predetermined to promote biological responses in the body lumen desired for certain clinical effectiveness. Further the coating can be used to mask (temporarily or permanently) the surface properties of the polymer used to comprise the stent embodiment. The coating can be selected from the broad class of any biocompatible bioresorbable polymer which can include any one or combination of halogenated and/or non-halogenated which can or cannot comprise any poly(alkylene glycol). These polymers can include compositional variations including homopolymers and heteropolymers, stereoisomers and/or a blend of such polymers. These polymers can include for example, but are not limited to, polycarbonates, polyarylates, poly(ester amides), poly(amide carbonates), trimethylene carbonate, polycaprolactone, polydioxane, polyhydroxybutyrate, poly-hydroxyvalerate, polyglycolide, polylactides and stereoisomers and copolymers thereof, such as glycolide/lactide copolymers.

In a preferred embodiment, the stent can be coated with a polymer that exhibits a negative charge that repels the negatively charged red blood cells' outer membranes thereby reducing the risk of clot formation. In another preferred embodiment, the stent can be coated with a polymer that exhibits an affinity for cells, (e.g., endothelial cells) to promote healing. In yet another preferred embodiment, the stent can be coated with a polymer that repels the attachment and/or proliferation of specific cells, for instance arterial fibroblasts and/or smooth muscle cells in order to lessen restenosis and/or inflammatory cells such as macrophages.

Described above are embodiments that can be modified with a coating to achieve functional properties that support biological responses. Such coatings or compositions of material with a therapeutic agent can be formed on stents or applied in the process of making a stent body via techniques such as dipping, spray coating, cross-linking combinations thereof, and the like, as mentioned and described above. Such coatings or compositions of material can also serve purpose other than delivering a therapeutic, such as to enhance stent retention on a balloon when the coating is placed intraluminally on the stent body and/or placed over the entire device after the stent is mounted on the balloon system to keep the stent in a compacted formation. Other purposes can be envisioned by those skilled in the art when using any polymer material.

In one aspect of certain embodiments, a stent would have a coating applied that can alter the physical characteristics of the stent, such as to provide specific mechanical properties to the stent. The properties can include *inter alia* thickness, tensile strength, glass transition temperature, and surface finish. The coating can be preferably applied prior to final crimping or application of the stent to the catheter. The stent can then be applied to the catheter and the system can have either heat or pressure or both applied in a compressive manner. In the process, the coating can form frangible bonds with both the catheter and the other stent surfaces. The bonds would enable a reliable method of creating stent retention and of holding the stent crossing profile over time. The bonds would break upon the balloon deployment pressures. The coating would be a lower Tg than the substrate to ensure no changes in the substrate.

### Stent Deployment

First, a catheter is provided wherein an expandable member, preferably an inflatable balloon, such as an angioplasty balloon, is provided along a distal end portion. One example of a balloon catheter for use with a stent is described in US Patent No. 4,733,665 to Palmaz. A stent on a catheter can be commonly collectively referred to as a stent system. Catheters include but are not limited to over-the-wire catheters, coaxial rapid-exchange designs and the Medtronic Zipper Technology that is a new delivery platform. Such catheters can include for instance those described in Bonzel US Patent Nos. 4,762,129 and 5,232,445 and by Yock US Patent Nos. 4,748,982, 5,496,346, 5,626,600, 5,040,548, 5,061,273, 5,350,395, 5,451,233 and 5,749,888. Additionally, catheters can include for instance those as described in US Patent Nos. 4,762,129, 5,092,877, 5,108,416, 5,197,978, 5,232,445, 5,300,085, 5,445,646, 5,496,275, 5,545,135, 5,545,138, 5,549,556, 5,755,708, 5,769,868, 5,800,393, 5,836,965, 5,989,280, 6,019,785, 6,036,715, 5,242,399, 5,158,548, and 6,007,545.

Catheters can be specialized with highly compliant polymers and for various purposes such as to produce an ultrasound effect, electric field, magnetic field, light and/or temperature effect. Heating catheters can include for example those described in US Patent Nos. 5,151,100, 5,230,349, 6,447,508, and 6,562,021, as well as International Publication No. WO9014046A1. Infrared light emitting catheters can include for example those described in US Patent Nos. 5,910,816 and 5,423,321.

An expandable member, such as an inflatable balloon, can be preferably used to deploy the stent at the treatment site. As the balloon is expanded, the radial force of the balloon overcomes the initial resistance of the constraining mechanism, thereby allowing the stent to expand.

The stent of embodiments described herein can be adapted for deployment using conventional methods known in the art and employing percutaneous transluminal catheter devices. This can include deployment in a body lumen by means of a balloon expandable design whereby expansion can be driven by the balloon expanding. Alternatively, the stent can be mounted onto a catheter that holds the stent as it is delivered through the body lumen and then releases the stent and allows it to self-expand into contact with the body lumen. The restraining means can comprise a removable/retractable sheath, a sheath that remains with the stent, and/or a mechanical aspect of the stent design.

The use of a sheath can be beneficial for several reasons. The sheath can be used to control delivery and deployment of the stent. For example, the sheath can be used to reduce and/or eliminate "negative aspects" of certain configurations of the stent, such as certain "slide-and-lock" designs; however, the sheath can also be used to make other designs possible.

Certain embodiments of the sheath are made of a polymeric material, such as a biodegradable material, which has sufficient elasticity to stretch during deployment of the stent and not break. The polymer also may include radiopaque, biodegradable polymers. The sheath is tubular in nature, and may include cutouts patterns to provide lower deployment pressures, increase flexibility and allow access to side branches of the artery. Ideally the sheath is very thin, such as less than 0.002", and ideality 0.0005" thick. The material need not have a high yield strength, but should have an elongation at break of greater than 150%, and possibly as much as 300%.

The sheath can be made from a variety of materials, such as polymers, natural materials, etc., which can include biodegradable materials. Further, the polymer can be radiopaque, biocompatible, and/or biodegradable, as discussed herein. In some embodiments, the sheath can be made from a resorbable material, and the sheath and stent can degrade together, thus leaving a healed, unencumbered vessel. The sheath material can be selected such that during stent expansion, the sheath can deform and expand plastically with the stent. In some embodiments, the sheath can have sufficient elasticity to stretch during deployment of the stent without breaking. Although high yield strength may not be required, the material preferably provides the sheath with an elongation at break of greater than 150%, and possibly as much as 300%.

Further, the sheath can be very thin, such as less than about 0.002 inches (0.0508 mm) thick, but can preferably be about 0.0005 inches (0.0127 mm) thick; other thicknesses can also be used in accordance with the teachings herein. Thus, the sheath can be beneficially used to eliminate or reduce negative aspects of certain stent designs, such as may be encountered during stent deployment, as well as to make other stent designs possible.

### Summary

From the foregoing description, it will be appreciated that a novel approach for expanding a lumen has been disclosed. While several components, techniques and aspects have been described with a certain degree of particularity, it is manifest that many changes can be made in the specific designs, constructions and methodology herein above described without departing from the scope of this disclosure. Indeed, the scope of this disclosure extends beyond the specifically disclosed stent embodiments to other alternative embodiments and/or uses of the embodiments and certain modifications and equivalents thereof. Various features and aspects of the disclosed stent embodiments can be combined with or substituted for one another in order to form varying modes of the conveyor. The scope of this disclosure should not be limited by the particular disclosed embodiments described herein.

Certain features that are described in this disclosure in the context of separate implementations can also be implemented in combination in a single implementation. Conversely, various features that are described in the context of a single implementation can also be implemented in multiple implementations separately or in any suitable subcombination. Moreover, although features may be described above as acting in certain combinations, one or more features from a claimed combination can, in some cases, be excised from the combination, and the combination may be claimed as any subcombination or variation of any subcombination.

The methods which are described and illustrated herein are not limited to the sequence of acts described, nor are they necessarily limited to the practice of all of the acts set forth. Other sequences of acts, or less than all of the acts, or simultaneous occurrence of the acts, can be utilized in practicing embodiments.

While a number of preferred embodiments and variations thereof have been described in detail, other modifications and methods of using and medical applications for the same will be apparent to those of skill in the art. Accordingly, it should be understood that various applications, modifications, materials, and substitutions can be made of equivalents without departing from the scope of the claims.

Various modifications and applications of the embodiments can occur to those who are skilled in the art. The present disclosure is not limited to the embodiments set forth herein for purposes of exemplification, but is to be defined only by a fair reading of the claims, including the full range of equivalency to which each element thereof is entitled.

For example, a uniform expandable stent can be provided that comprises a tubular member having a circumference which is expandable between at least a first compacted diameter and at least a second expanded diameter, said tubular member comprising; at least two slideably engaged radial elements collectively defining the circumference of said tubular member, said at least two slideably engaged radial elements individually comprising; a flexible backbone, a first elongate member and a second elongate member, wherein said first elongate member and said second elongate member are substantially commonly oriented with respect to said flexible backbone, wherein said second elongate member is at least partially circumferentially-offset with respect to said first elongate member.

In some embodiments, at least one of said first elongate member and said second elongate member can be a paired elongate member. In some embodiments, at least one of said first elongate member or said second elongate member can be an annular elongate member. In some embodiments, said annular elongate member can further comprise a substantially captive slot. Further, said flexible backbone can be configured to substantially coil about said tubular member.

Moreover, said flexible backbone can be configured to stair-step in a helical orientation about said tubular member. In this regard, said flexible backbone can further comprise at least one substantially captive slot. Said substantially captive slot can further comprise a locking member. Said locking member can further comprise at least one of a tooth, a deflectable tooth, or a stop. In some embodiments, at least one of said elongate members can comprise at least one conjugate locking member, wherein said locking member and said conjugate locking member define an engagement means, said engagement means being adapted to allow substantially unidirectional slideable movement. At least one of said elongate members can further comprise a first axial side and a second axial side, wherein at least one of said first axial side and said second axial side comprises at least one conjugate locking member. Said at least one conjugate locking member is one of a tooth, a deflectable tooth, or a stop.

Terms of orientation used herein, such as "top," "bottom," "horizontal," "vertical," "longitudinal," "lateral," and "end" are used in the context of the illustrated embodiment. However, the present disclosure should not be limited to the illustrated orientation. Indeed, other orientations are possible and are within the scope of this disclosure. Terms relating to circular shapes as used herein, such as diameter or radius, should be understood not to require perfect circular structures, but rather should be applied to any suitable structure with a cross-sectional region that can be measured from side-to-side. Terms relating to shapes generally, such as "circular" or "cylindrical" or "semicircular" or "semi-cylindrical" or any related or similar terms, are not required to conform strictly to the mathematical definitions of circles or cylinders or other structures, but can encompass structures that are reasonably close approximations.

Conditional language, such as "can," "could," "might," or "may," unless specifically stated otherwise, or otherwise understood within the context as used, is generally intended to convey that certain embodiments include or do not include, certain features, elements, and/or steps. Thus, such conditional language is not generally intended to imply that features, elements, and/or steps are in any way required for one or more embodiments.

Conjunctive language, such as the phrase "at least one of X, Y, and Z," unless specifically stated otherwise, is otherwise understood with the context as used in general to convey that an item, term, etc. may be either X, Y, or Z. Thus, such conjunctive language is not generally intended to imply that certain embodiments require the presence of at least one of X, at least one of Y, and at least one of Z.

The terms "approximately," "about," and "substantially" as used herein represent an amount close to the stated amount that still performs a desired function or achieves a desired result. For example, in some embodiments, as the context may dictate, the terms "approximately", "about", and "substantially" may refer to an amount that is within less than or equal to 10% of the stated amount. The term "generally" as used herein represents a value, amount, or characteristic that predominantly includes or tends toward a particular value, amount, or characteristic. As an example, in certain embodiments, as the context may dictate, the term "generally parallel" can refer to something that departs from exactly parallel by less than or equal to 20 degrees.

Some embodiments have been described in connection with the accompanying drawings. The figures are drawn to scale, but such scale should not be limiting, since dimensions and proportions other than what are shown are contemplated and are within the scope of the disclosed invention. Distances, angles, etc. are merely illustrative and do not necessarily bear an exact relationship to actual dimensions and layout of the devices illustrated. Components can be added, removed, and/or rearranged. Further, the disclosure herein of any particular feature, aspect, method, property, characteristic, quality, attribute, element, or the like in connection with various embodiments can be used in all other embodiments set forth herein. Additionally, it will be recognized that any methods described herein may be practiced using any device suitable for performing the recited steps.

In summary, various embodiments and examples of stents have been disclosed. Although the stents have been disclosed in the context of those embodiments and examples, this disclosure extends beyond the specifically disclosed embodiments to other alternative embodiments and/or other uses of the embodiments, as well as to certain modifications and equivalents thereof. This disclosure expressly contemplates that various features and aspects of the disclosed embodiments can be combined with, or substituted for, one another. Accordingly, the scope of this disclosure should not be limited by the particular disclosed embodiments described above, but should be determined only by a fair reading of the claims that follow.

### References

Some of the references cited herein are listed below, the entirety of each one of:
Charles R, Sandirasegarane L, Yun J, Bourbon N, Wilson R, Rothstein RP, et al., Ceramide-Coated Balloon Catheters Limit Neointimal Hyperplasia after Stretch Injury in Carotid Arteries, Circulation Research 2000; 87(4): 282-288.
Coroneos E, Martinez M, McKenna S, Kester M., Differential regulation of sphingomyelinase and ceramidase activities by growth factors and cytokines. Implications for cellular proliferation and differentiation, J Biol. Chem. 1995; 270(40): 23305-9.
Coroneos E, Wang Y, Panuska JR, Templeton DJ, Kester M., Sphingolipid metabolites differentially regulate extracellular signal-regulated kinase and stress-activated protein kinase cascades, Biochem J. 1996; 316 (Pt 1): 13-7.
Jacobs LS, Kester M., Sphingolipids as mediators of effects of platelet-derived growth factor in vascular smooth muscle cells, Am. J. Physiology 1993; 265 (3 Pt 1): C740-7.
Tanguay JF, Zidar JP, Phillips HR, 3rd, Stack RS, Current status of biodegradable stents, Cardiol. Clin. 1994; 12(4): 699-713.
Nikol S, Huehns TY, Hofling B., Molecular biology and post-angioplasty restenosis, Atherosclerosis 1996; 123 (1-2): 17-31.
BUDDY D. RATNER, ALLAN S. HOFFMAN, FREDERICK J. SCHOEN, AND JACK E. LEMONS, Biomaterials Science: An Introduction to Materials in Medicine (Elsevier Academic Press 2004).

## Claims

1. A radially expandable stent (10, 20) having longitudinal and circumferential axes, the stent comprising:
a plurality of rings arranged longitudinally to form a tubular body assembly, each ring comprising a circumferential sequence of cells (14, 21), wherein:
each cell is longitudinally defined by a first pair of deformable struts (12, 22) on a first longitudinal side and a second pair of deformable struts (12, 22) on a second longitudinal side, the struts of the first pair set at an angle to one another and the struts of the second pair set at an angle to one another, the angle between each pair increasing as the stent is expanded from a compact configuration towards an expanded configuration; and
at least one cell is circumferentially defined by a first cross member (24) and a second cross member (25), the first cross member fixedly supporting a distal end (31) of a rail member (26), the second cross member slidingly engaging a medial portion (33) of the rail member;
wherein the rail member is configured to slide circumferentially in a first direction with respect to the second cross member as the stent is expanded towards an expanded configuration; and
wherein the medial portion of the rail member includes at least one tooth element configured to engage the second cross member so as to permit sliding in the first direction, and to engage the second cross member so as to resist sliding in an opposite second direction, so as to inhibit the stent from radially contracting.

2. The stent of Claim 1, wherein each cell is circumferentially defined by a first cross member and a second cross member, the first cross member fixedly supporting a distal end of a rail member, the second cross member slidingly engaging a medial portion of the rail member.

3. The stent of Claim 1, further comprising link elements connecting longitudinally adjacent rings.

4. The stent of Claim 1, wherein longitudinally adjacent rings comprise integrated portions.

5. The stent of Claim 1, wherein each of the rings has a substantially zig-zag shape comprising a plurality of peaks and a plurality of valleys.

6. The stent of Claim 1, wherein the rings are arranged in a peak-to-peak configuration.

7. The stent of Claim 5, wherein a first ring of the plurality of rings is about 180 degrees out of phase from a second ring of the plurality of rings.

8. The stent of Claim 5, wherein the first cross member connects one of the plurality of peaks of a first ring with one of the plurality of peaks of a second ring.

9. The stent of Claim 1, wherein the medial portion of the rail member comprises a plurality of teeth.

10. The stent of Claim 1, wherein the second cross member comprises a channel comprising a closed aperture.

11. The stent of Claim 1, wherein the second cross member comprises a channel open on a radial side.

12. The stent of Claim 5, wherein the plurality of peaks and the plurality of valleys are substantially flat.

13. The stent of Claim 1, wherein the struts are configured to rotate with respect to the longitudinal axis.

14. The stent of Claim 1, wherein:
when the stent is in the compact configuration, and when the stent is in the expanded configuration, the struts are not parallel with the longitudinal axis; and
when the stent is in an intermediate configuration, between the compact configuration and the expanded configuration, the struts are substantially parallel with the longitudinal axis.

15. The stent of Claim 1, wherein the rail member has a substantially linear shape.

## Patentansprüche

1. Radial dehnbarer Stent (10, 20) mit längslaufenden und umlaufenden Achsen, wobei der Stent Folgendes umfasst:
eine Mehrheit von in Längsrichtung angeordneten Ringen zur Bildung einer rohrförmigen Körpereinheit, wobei jeder Ring eine umlaufende Sequenz von Zellen (14, 21) umfasst, wobei:
jede Zelle in Längsrichtung durch ein erstes Paar von verformbaren Streben (12, 22) an einer ersten längslaufenden Seite und ein zweites Paar von verformbaren Streben (12, 22) an einer zweiten längslaufenden Seite definiert ist, wobei die Streben des ersten Paars in einem Winkel zueinander eingestellt ist und die Streben des zweiten Paars in einem Winkel zueinander eingestellt ist, wobei sich der Winkel zwischen jedem Paar vergrößert, wenn der Stent von einer kompakten Gestaltung gegen eine ausgedehnte Gestaltung ausgedehnt wird; und
mindestens eine Zelle umlaufend durch ein erstes Querelement (24) und ein zweites Querelement (25) definiert ist, wobei das erste Querelement ein distales Ende (31) eines Bahnelements (26) fest unterstützt, und das zweite Querelement in einen Mittelteil (33) des Bahnelements gleitend eingreift;
wobei das Bahnelement dafür ausgelegt ist, in einer ersten Richtung im Verhältnis zum zweiten Querelement umlaufend zu gleiten, wenn der Stent gegen eine ausgedehnte Gestaltung ausgedehnt wird; und
wobei der Mittelteil des Bahnelements mindestens ein Zahnelement umfasst, das dafür ausgelegt ist, in das zweite Querelement einzugreifen, um ein Gleiten in die erste Richtung zu ermöglichen, und in das zweite Querelement einzugreifen, um ein Gleiten in eine entgegengesetzte zweite Richtung zu widerstehen, um zu verhindern, dass sich der Stent radial zusammenzieht.

2. Stent nach Anspruch 1, wobei jede Zelle umlaufend durch ein erstes Querelement und ein zweites Querelement definiert ist, wobei das erste Querelement ein distales Ende eines Bahnelements fest unterstützt, und das zweite Querelement in einen Mittelteil des Bahnelements gleitend eingreift.

3. Stent nach Anspruch 1, weiter umfassend Verknüpfungselemente, die längslaufende benachbarte Ringe verbinden.

4. Stent nach Anspruch 1, wobei längslaufende benachbarte Ringe integrierte Teile umfassen.

5. Stent nach Anspruch 1, wobei jeder der Ringe eine im Wesentlichen zickzackförmige Form aufweist, umfassend eine Mehrheit von Spitzen und eine Mehrheit von Vertiefungen.

6. Stent nach Anspruch 1, wobei die Ringe in einer Spitze-an-Spitze-Gestaltung angeordnet sind.

7. Stent nach Anspruch 5, wobei ein erster Ring der Mehrheit von Ringen ca. 180 Grad von einem zweiten Ring der Mehrheit von Ringen phasenverschoben ist.

8. Stent nach Anspruch 5, wobei das erste Querelement eine der Mehrheit von Spitzen eines ersten Rings mit einer der Mehrheit von Spitzen eines zweiten Rings verbindet.

9. Stent nach Anspruch 1, wobei der Mittelteil des Bahnelements eine Mehrheit von Zähnen umfasst.

10. Stent nach Anspruch 1, wobei das zweite Querelement einen Kanal mit einer geschlossenen Öffnung umfasst.

11. Stent nach Anspruch 1, wobei das zweite Querelement einen Kanal umfasst, der an einer radialen Seite offen ist.

12. Stent nach Anspruch 5, wobei die Mehrheit von Spitzen und die Mehrheit von Vertiefungen im Wesentlichen flach sind.

13. Stent nach Anspruch 1, wobei die Streben dafür ausgelegt sind, im Verhältnis zur Längsachse zu drehen.

14. Stent nach Anspruch 1, wobei:
wenn der Stent in der kompakten Gestaltung ist, und wenn der Stent in der ausgedehnten Gestaltung ist, sind die Streben nicht parallel zur Längsachse; und
wenn der Stent in der zwischenliegenden Gestaltung ist, zwischen der kompakten Gestaltung und der ausgedehnten Gestaltung, sind die Streben im Wesentlichen parallel zur Längsachse.

15. Stent nach Anspruch 1, wobei das Bahnelement eine im Wesentlichen lineare Form aufweist.

## Revendications

1. Endoprothèse dilatable radialement (10, 20) ayant des axes longitudinaux et circonférentiels, l'endoprothèse comprenant :
une pluralité d'anneaux agencés longitudinalement pour former un ensemble de corps tubulaire, chaque anneau comprenant une séquence de cellules circonférentielle (14, 21), dans laquelle :
chaque cellule est définie longitudinalement par une première paire d'entretoises déformables (12, 22) sur un premier côté longitudinal et une deuxième paire d'entretoises déformables (12, 22) sur un deuxième côté longitudinal, les entretoises de la première paire formant un angle entre elles et les entretoises de la deuxième paire formant un angle entre elles, l'angle entre chaque paire augmentant à mesure que l'endoprothèse est étendue d'une configuration compacte à une configuration étendue ; et
au moins une cellule est définie de manière circonférentielle par un premier élément transversal (24) et un deuxième élément transversal (25), le premier élément transversal supportant de manière fixe une extrémité distale (31) d'un élément de rail (26), le deuxième élément transversal venant en prise de manière coulissante avec une partie médiane (33) de l'élément de rail ;
dans laquelle l'élément de rail est configuré de manière à coulisser de façon circonférentielle dans une première direction par rapport au deuxième élément transversal lorsque l'endoprothèse est étendue vers une configuration étendue ; et
dans lequel la partie médiane de l'élément de rail comprend au moins un élément de dent configuré pour engager le deuxième élément transversal de manière à permettre un glissement dans la première direction, et pour engager le deuxième élément transversal de manière à résister au glissement dans une deuxième direction opposée, afin d'empêcher l'endoprothèse de se contracter radialement.

2. Endoprothèse selon la revendication 1, dans laquelle chaque cellule est définie de manière circonférentielle par un premier élément transversal et un deuxième élément transversal, le premier élément transversal supportant de manière fixe une extrémité distale d'un élément de rail, le deuxième élément transversal venant en prise de manière coulissante avec une partie médiane de l'élément de rail.

3. Endoprothèse selon la revendication 1, comprenant en outre des éléments de liaison reliant des anneaux adjacents longitudinalement.

4. Endoprothèse selon la revendication 1, dans laquelle des anneaux adjacents longitudinalement comprennent des parties intégrées.

5. Endoprothèse selon la revendication 1, dans laquelle chacun des anneaux a une forme essentiellement en zigzag comprenant une pluralité de sommets et une pluralité de vallées.

6. Endoprothèse selon la revendication 1, dans laquelle les anneaux sont agencés dans une configuration pic à pic.

7. Endoprothèse selon la revendication 5, dans laquelle un premier anneau de la pluralité d'anneaux est déphasé d'environ 180 degrés par rapport à un deuxième anneau de la pluralité d'anneaux.

8. Endoprothèse selon la revendication 5, dans laquelle le premier élément transversal relie l'un de la pluralité de sommets d'un premier anneau à l'un de la pluralité de sommets d'un deuxième anneau.

9. Endoprothèse selon la revendication 1, dans laquelle la partie médiane de l'élément de rail comprend une pluralité de dents.

10. Endoprothèse selon la revendication 1, dans laquelle le deuxième élément transversal comprend un canal comprenant une ouverture fermée.

11. Endoprothèse selon la revendication 1, dans laquelle le deuxième élément transversal comprend un canal ouvert sur un côté radial.

12. Endoprothèse selon la revendication 5, dans laquelle la pluralité de sommets et la pluralité de vallées sont essentiellement plates.

13. Endoprothèse selon la revendication 1, dans laquelle les entretoises sont configurées de manière à tourner par rapport à l'axe longitudinal.

14. Endoprothèse selon la revendication 1, dans laquelle :
lorsque l'endoprothèse est dans la configuration compacte et lorsque l'endoprothèse est dans la configuration étendue, les entretoises ne sont pas parallèles à l'axe longitudinal ; et
lorsque l'endoprothèse est dans une configuration intermédiaire, entre la configuration compacte et la configuration étendue, les entretoises sont essentiellement parallèles à l'axe longitudinal.

15. Endoprothèse selon la revendication 1, dans laquelle l'élément de rail a une forme essentiellement linéaire.
